# EUROPÄISCHE PATENTSCHRIFT

(11) **EP 3 858 297 B1**
(45) Veröffentlichungstag und Bekanntmachung des Hinweises auf die Patenterteilung: **13.07.2022**
(21) Anmeldenummer: 21160938.3
(22) Anmeldetag: 19.12.2018
(51) Int. Cl.: A61F 2/50, A61F 2/60, A61F 2/64, A61F 2/68, A61F 2/70, A61F 5/01

(54) **ORTHOPÄDIETECHNISCHE EINRICHTUNG**
ORTHOPEDIC DEVICE
DISPOSITIF TECHNIQUE ORTHOPÉDIQUE

(30) Priorität: 27.12.2017 DE 102017131319
(43) Veröffentlichungstag der Anmeldung: 04.08.2021
(62) Teilanmeldung aus: 18827048.2
(73) Patentinhaber: Otto Bock Healthcare Products GmbH, 1110 Wien (AT)
(72) Erfinder: AUBERGER, Roland, 1140 Wien (AT); BREUER-RUESCH, Christian, 1230 Wien (AT); SPRING, Alexander,Noah, Ottawa, Ontario, K1Y 1W3 (CA); SEIFERT, Dirk, 1070 Wien (AT); VOLKMAR, Marco, 37115 Duderstadt (DE)
(74) Vertreter: Gramm, Lins & Partner Patent- und Rechtsanwälte PartGmbB

(56) Entgegenhaltungen:
- WO-A1-2008/103917
- WO-A1-2016/169855
- WO-A2-2009/140956
- DE-A1-102014 009 028
- DE-B3-102013 013 810

## Beschreibung

Die Erfindung betrifft eine orthopädietechnische Einrichtung, insbesondere Orthese, oder Exoskelett, mit einem Oberteil und einem Unterteil, die über eine Gelenkeinrichtung schwenkbar um eine Gelenkachse miteinander verbunden sind und Befestigungseinrichtungen aufweisen, mit denen die orthopädietechnische Einrichtung, z.B. Exoskelette oder Orthesen an einer Gliedmaße festlegbar ist, und mit einem Aktuator, der an dem Oberteil und dem Unterteil an Befestigungsstellen festgelegt ist und eine Verschwenkung des Oberteils relativ zu dem Unterteil beeinflusst.

Orthesen oder Exoskelette, die nachfolgend als Orthesen zusammengefasst werden, werden unter anderem dazu eingesetzt, um die Funktion einer noch vorhandenen Extremität oder Gliedmaße zu unterstützen oder aufrechtzuerhalten. Dazu wird die Orthese an der Gliedmaße festgelegt. Bei einer Orthese der unteren Extremität, beispielsweise einer das Kniegelenk übergreifenden Orthese, werden an dem Oberschenkel und an dem Unterschenkel Oberteile und Unterteile oder Schienen angelegt, die über eine Gelenkeinrichtung, das Orthesenkniegelenk, miteinander verbunden werden. Bei einer Knöchelorthese wird der Fuß auf einem Fußteil fixiert, das über ein Orthesenknöchelgelenk mit einem Unterschenkelteil, das seinerseits als Oberteil dient, verbunden ist. Entsprechendes gilt für eine Hüftgelenksorthese oder für eine Orthese, die mehr als zwei natürliche Gelenke überbrückt. Ebenfalls existieren Orthesen für obere Extremitäten, beispielsweise eine Orthese, die das Ellenbogengelenk überbrückt. Als Oberteil wird jeweils die proximale Komponente einer Orthese angesehen, die mit einer distalen Komponente als Unterteil über die Gelenkeinrichtung verbunden ist. Als Befestigungseinrichtungen dienen Schalen, Gurte, Klammern oder andere Einrichtungen, mit denen verhindert wird, dass die Gliedmaße sich von der Orthese löst.

Prothesen dienen als Ersatz einer nicht vorhandenen Gliedmaße und sind häufig mit einem Prothesengelenk ausgestattet, das zwei Prothesenkomponenten miteinander schwenkbar verbindet. Um die Bewegung der Komponenten zueinander zu beeinflussen, werden Aktuatoren, z.B. Dämpfer, Bremsen und/oder Antriebe, eingesetzt, die gesteuert oder ungesteuert Verschwenkbewegungen beeinflussen, insbesondere dämpfen oder unterstützen.

Um die Bewegungen des Oberteils und Unterteils zueinander während der Verschwenkbewegung zu beeinflussen, beispielsweise um die Verschwenkbewegung zu unterstützen oder zu behindern, werden Aktuatoren eingesetzt, die an dem Oberteil und dem Unterteil befestigt sind. Eine Bewegungsunterstützung kann über die Zufuhr von Energie aus einem Energiespeicher bei einer Ausgestaltung des Aktuators als Antrieb erfolgen. Als Energiespeicher können Federeinrichtungen oder Speicher elektrischer Energie zum Antreiben eines Motors oder einer Pumpe vorgesehen sein. Rein passive Aktuatoren sind Dämpfer oder Bremsen, die einen einstellbaren Widerstand gegen die Verschwenkbewegung bereitstellen. Die Dämpfung kann sowohl hinsichtlich Flexionsrichtung als auch Extensionsrichtung eingesetzt werden. Es sind Anschlagelemente in der jeweiligen Gelenkeinrichtung oder in dem Aktuator vorhanden, um bei Erreichen einer Maximalstellung des Oberteils relativ zu dem Unterteil eine weitere Bewegung zu vermeiden und einen Anschlag zu gewährleisten. Die Anschlagelemente können sowohl für eine Flexionsrichtung, bei der ein Gelenkwinkel verkleinert wird, als auch für die Extensionsrichtung, bei der der Gelenkwinkel vergrößert wird, vorgesehen sein. Der maximale Extensionswinkel ist in der Regel erreicht, wenn zwischen der Längsorientierung des Oberteils und des Unterteils ein Winkel von 180° besteht. Der damit korrelierende Gelenkwinkel ergibt sich aus der für den Körper des Patienten aufgrund der anatomischen und physiologischen Gegebenheiten maximalen Streckstellung. Diese kann auch eine meist pathologische Hyperextensionsstellung darstellen, wodurch der Gelenkwinkel über 180° betragen kann. Eine Hyperextension kann beispielsweise bei einem Kniegelenk auftreten, bei dem der Winkel zwischen der Rückseite der Wade und der Rückseite des Oberschenkels größer als 180° sein kann. Entsprechend tritt eine Hyperextension bei einem Ellenbogengelenk auf, wenn zwischen dem Unterarm und der Trizepsseite des Oberarmes ein Winkel kleiner als 180° auftritt.

Aus der WO 2009/097841 A1 ist ein orthopädietechnisches Kniegelenk mit einem Oberteil, an dem obere Anschlussmittel angeordnet sind, und einem verschwenkbar an dem Oberteil gelagerten Unterteil mit Anschlussmitteln für orthopädische Komponenten bekannt, das einen Anschlag zur Begrenzung einer Extensionsbewegung aufweist. Der Anschlag ist verlagerbar ausgebildet und mit einer Verstelleinrichtung gekoppelt, die wiederum mit einer Steuereinrichtung gekoppelt ist, die in Abhängigkeit von Sensordaten die Verstelleinrichtung aktuiert und die Position des Anschlages verändert. Das orthopädische Kniegelenk kann auch als ein Orthesenkniegelenk ausgebildet sein. Die Anmeldung betrifft der Anpassung der Lage des Extensionsanschlages an unterschiedliche momentane oder kurzfristige Anforderungen während des Gehens und Stehens. Eine Anpassung an die anatomischen Voraussetzungen des Anwenders ist nicht offenbart.

Die Position des Extensionsanschlages einer Orthese muss an die anatomischen und physiologischen Gegebenheiten des jeweiligen Anwenders angepasst werden. Neben statischen Extensionsanschlägen, die bei dem Anpassen der Orthese an den Patienten einmalig eingestellt werden und bis zu einer erneuten Einstellung durch einen Orthopädietechniker nicht verändert werden, hängt die Qualität der Einstellung des Extensionsanschlages von der Erfahrung des Orthopädietechnikers ab. Bei motorisch verstellbaren Extensionsanschlagspositionen wird die Einstellbarkeit erleichtert, die einzelnen Positionen selbst müssen in der Steuerung abgelegt oder von dem Orthopädietechniker eingegeben werden. Die Korrektur von Extensionsanschlagspositionen kann beispielsweise notwendig sein, um Fertigungsungenauigkeiten korrigieren zu können oder um Beugekontrakturen bei pathologischen Zuständen Rechnung zu tragen. Diese Kontrakturen können sich im Lauf der Zeit ändern, beispielsweise als Reaktion auf eine Therapie. Es können sich auch Änderungen im Verlauf eines Tages aufgrund der Aktivitäten des Patienten einstellen. Daher kann eine Korrektur der Extensionsanschlagsposition oder der Ausrichtung der Gliedmaße an dem Oberteil relativ zu dem Unterteil notwendig sein, um Änderungen während der täglichen Tragdauer vorzunehmen oder um auf unterschiedliche Bewegungssituationen zu reagieren.

Die WO 2008/103917 A1 betrifft einen Prothesenfuß, der gelenkig an einem Prothesenunterschenkel angeordnet wird. Zwischen dem Prothesenunterschenkel und dem Prothesenfuß sind Hydraulikdämpfer mit Hydraulikzylindern mit darin angeordneten Kolben angeordnet. Ein erster Kolben sorgt für die gedämpfte Bewegung des Knöchelgelenkes, während ein zweiter Kolben verwendet wird, um eine Absatzhöhe einzustellen. Nachdem die Absatzhöhe eingestellt ist, wird der zweite Kolben gesperrt, sodass eine weitere Bewegung dieses Kolbens nicht möglich ist.

Aufgabe der vorliegenden Erfindung ist es, eine orthopädietechnische Einrichtung bereitzustellen, mit denen auf einfache Art und Weise der Tragekomfort für den Nutzer der Orthese und eine Anpassung an sich verändernde anatomische und physiologische Gegebenheiten erzielen lässt.

Erfindungsgemäß wird diese Aufgabe durch eine orthopädietechnische Einrichtung mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Die erfindungsgemäße orthopädietechnische Einrichtung mit einem Oberteil und einem Unterteil, die über eine Gelenkeinrichtung schwenkbar um eine Gelenkachse miteinander verbunden sind und Befestigungseinrichtungen aufweisen, mit denen die orthopädietechnische Einrichtung an einer Gliedmaße festlegbar ist, und mit einem Aktuator, der an dem Oberteil und dem Unterteil an Befestigungsstellen festgelegt ist und eine Verschwenkung des Oberteils relativ zu dem Unterteil beeinflusst sieht vor, dass die Orientierung des Unterteils relativ zu der an dem Oberteil festlegbaren Gliedmaße einstellbar ist. Neben einer Änderung der Orientierung des Oberteils relativ zu dem Unterteil um die Schwenkachse der Gelenkeinrichtung durch eine Veränderung der Endanschläge der Gelenkeinrichtung und damit der Veränderung der Endlagen des Oberteils und der Unterteils zueinander ist es möglich, die Orientierung der Gliedmaße an oder in dem Oberteil oder Unterteil relativ zu dem Oberteil oder Unterteil zu verändern. Dadurch kann bei einer Ausgestaltung der orthopädietechnischen Einrichtung als Orthese der maximal erreichbare Extensionswinkel und/oder Flexionswinkel der Gliedmaße festgelegt werden wiederholt verändert werden, ohne die Gelenkeinrichtung oder den Aktuator zu manipulieren. Bei einer Ausgestaltung der orthopädietechnischen Einrichtung als Prothese kann die Orientierung des Stumpfes in dem oder an dem Oberteil verändert werden oder der Stumpf in unterschiedlichen Orientierungen in dem Oberteil, z.B. einem Prothesenschaft, eingesetzt werden, wodurch sich die Ausrichtung des Unterteil zu dem Stumpf verändert und in der jeweiligen Stellung festgelegt werden kann. Eingriffe an dem Prothesengelenk sind dazu nicht notwendig. Alternativ oder ergänzend kann die Orientierung durch Veränderungen an oder in dem Aktuator verändert werden, indem der maximal erreichbare Winkel in Flexionsrichtung und/oder Extensionsrichtung durch eine Veränderung des maximalen Bewegungsumfanges des Aktuators in die eine oder andere Richtung begrenzt und verändert wird. Alternativ oder ergänzend kann die Orientierung des Unterteils relativ zu dem Oberteil durch verstellbare Anschläge oder Begrenzungen an dem Oberteil und/oder Unterteil oder der Gelenkeinrichtung erreicht werden, beispielsweise durch mechanische Anschläge, hydraulische Anschläge oder andere Einstell- oder Verstellmechanismen. Die Befestigungsstelle kann verschieblich oder verlagerbar, z.B. verdrehbar an dem Oberteil bzw. dem Unterteil oder einer kraftübertragenden Schnittstelle der Gelenkeinrichtung gelagert sein. Die Verschieblichkeit kann insbesondere geradlinig sein, beispielsweise in einer geraden Führungsschiene oder Nut. Die Führungsschiene oder die Führungsnut kann auch eine gekrümmte oder anderweitige Form aufweisen, so dass sie als eine Kulissenführung mit einer vorgegebenen Bewegungsbahn der Befestigungsstelle ausgebildet sein kann. Darüber hinaus kann die Befestigungsstelle verdrehbar an dem Oberteil bzw. Unterteil gelagert sein, wobei durch eine exzentrische Lagerung die Veränderung des Abstandes der Befestigungsstelle zu der Gelenkachse erfolgt. Die Verdrehbarkeit kann in Rastungen oder auch stufenlos erfolgen. Ebenso kann neben einer motorisch angetriebenen Verdrehung oder Verstellung über einen Motor die Verdrehung, Verschiebung oder Verstellung von einem Orthopädietechniker ausgeführt werden. Alternativ kann die Befestigungsstelle oder können die Befestigungsstellen an einer auswechselbaren Komponente angeordnet oder ausgebildet sein, um unterschiedliche Positionen der Befestigungsstelle oder Befestigungsstellen für die Gelenkeinrichtung zu realisieren. An den auswechselbaren Komponenten können an unterschiedlichen Stellen oder Positionen Einrichtungen zum Befestigen der Gelenkeinrichtung oder ggf. auch des Aktuators angeordnet sein, so dass je nach eingesetzter Komponente ein anderer Extensionsanschlag eingestellt wird. Jede Position der Befestigungseinrichtung bietet einen anderen Abstand und führt zu einem anderen Extensionsanschlag bei voller Ausnutzung des Extensionsweges für den jeweiligen Aktuator. Die Befestigungsstelle kann auch an einer auswechselbaren Komponente angeordnet oder ausgebildet sein, an der ein oder mehrere Befestigungseinrichtungen für die Gelenkeinrichtung oder den Aktuator angeordnet oder ausgebildet sind.

An der orthopädietechnischen Einrichtung kann ein einstellbarer Extensionsanschlag angeordnet oder ausgebildet und/oder eine auswechselbare oder einstellbare Anlage für die Gliedmaße angeordnet sein. Die Anlage kann als Polster, Einsatz, Kissen oder Einlage ausgebildet sein, die in ihrer Position und/oder in ihrem Volumen veränderbar sind, so dass sich eine veränderte Ausrichtung des Oberteils und/oder Unterteils relativ zu der Gliedmaße erreichen lässt. Die Anlage kann an einer Schiene, Schale oder einem Schaft angeordnet sein und über Einstelleinrichtungen relativ dazu verlagert werden. Kissen oder Polster können befüllt oder entleert werden, um Orientierungen zu verändern. An der orthopädietechnischen Einrichtung kann alternativ oder ergänzend ein einstellbarer Flexionsanschlag angeordnet oder ausgebildet sein, um eine mechanische oder elektronisch gesteuerte Limitierung der Verschwenkung der Gelenkeinrichtung zu erreichen. Der Extensionsanschlag ist in einer Variante der Erfindung an oder in dem Aktuator, an oder in einer kraftübertragenden Schnittstelle zwischen dem Aktuator und dem Oberteil und/oder Unterteil und/oder an oder in der zumindest einen Gelenkeinrichtung ausgebildet.

Eine Weiterbildung der Erfindung sieht vor, dass der Abstand zumindest einer Befestigungsstelle zu der Gelenkachse verstellbar ist. Mechanische Endanschläge an der Gelenkeinrichtung können vorgesehen sein, die beispielsweise über Schrauben oder unterschiedliche Einsätze verändert werden können, so dass sich unterschiedliche Gelenkwinkel zwischen dem Oberteil und dem Unterteil als maximaler Gelenkwinkel einstellen lässt. Ebenfalls oder alternativ ist innerhalb des Aktuators eine Verstellung des Extensionsanschlages durch elektronisch gesteuertes Öffnen und Schließen von Hydraulikleitungen vorgesehen oder aber eine Veränderung in dem Aktuator selbst, beispielsweise durch eine Veränderung der Länge des Gehäuses oder aber durch eine Verlängerung oder Verkürzung einer Kolbenstange über eine Gewindeverstellung. Eine elektronisch gesteuerte Verstellung des Extensionsanschlages, die als Variante der Erfindung vorgesehen ist, setzt eine leistungsfähige Steuerung voraus, die bei entsprechend ausgebildeten Orthesen oder Prothesen eingesetzt wird. Ein Eingriff in die Konstruktion und in die Abmessungen eines Aktuators erweist sich häufig als schwierig, da dann keine Standardaktuatoren, beispielsweise Standardhydraulikdämpfer oder Hydraulikantriebe, eingesetzt werden können. Daher ist die Veränderung des Abstandes zumindest einer der Befestigungsstellen des Aktuators an dem Oberteil oder dem Unterteil relativ zu der Gelenkachse eine einfache Art und Weise der Anpassung der orthopädietechnischen Einrichtung und des mit einem unveränderten Aktuator erreichbaren Extensionsanschlages. Der Extensionsanschlag wird innerhalb des Aktuators durch die Begrenzung beispielsweise des Verlagerungsweges eines Hydraulikkolbens innerhalb eines Zylinders bereitgestellt. Alternativ oder ergänzend kann der Extensionsanschlag innerhalb der Aktuators durch Begrenzung oder Veränderung des Verlagerungsweges eines Hydraulikkolbens innerhalb des Zylinders bereitgestellt werden. Der Extensionsanschlag kann ebenfalls durch geeignetes Schließen von Ventilen und/oder Sperren von Hydraulikleitungen eingestellt oder verstellt werden, wenn der Aktuator als Hydraulikaktuator ausgebildet ist.

Wird die Befestigungsstelle beispielsweise an dem Oberteil weiter proximal von der Gelenkachse verlagert, wird ein entsprechender Anschlag bei einem geringeren Gelenkwinkel erreicht, also vor Erreichen der maximal gestreckten Stellung. Wird die Befestigungsstelle weiter in Richtung auf die Gelenkachse verlagert, vergrößert sich der mögliche Gelenkwinkel, der Extensionsanschlag wird in Richtung auf eine zunehmende Extension oder auf einen maximalen Gelenkwinkel verstellt. Die Verstellung der Befestigungsstelle kann auf vielfältige Arten und Weisen erfolgen, insbesondere dadurch, dass zumindest eine der Befestigungsstellen verlagerbar an dem jeweils zugeordneten Oberteil oder Unterteil oder einer kraftübertragenden Schnittstelle gelagert ist. Neben einer nur an einer Stelle vorgesehenen Verlagerbarkeit ist es möglich, auch beide Befestigungsstellen verlagerbar auszugestalten, wobei die proximale Befestigungsstelle an dem Oberteil oder einer proximalen kraftübertragenden Schnittstelle verlagerbar oder relativ zu der Gelenkachse verstellbar ausgebildet ist und ebenso die distale Befestigungsstelle an dem Unterteil oder einer distalen kraftübertragenden Schnittstelle der Gelenkeinrichtung oder eines Gelenkmoduls verstellbar ausgebildet ist.

In einer Weiterbildung der Erfindung ist es vorgesehen, dass die Befestigungsstelle motorisch verstellbar an dem Oberteil bzw. dem Unterteil oder einer kraftübertragenden Schnittstelle gelagert ist, um bevorzugt stufenlos die Befestigungsstelle oder die Befestigungsstellen verlagern und damit den Extensionsanschlag verstellen zu können.

Eine Weiterbildung der Erfindung sieht vor, dass die Befestigungsstelle an einem Adapter angeordnet oder ausgebildet ist, an dem mehrere Befestigungseinrichtungen für den Aktuator und/oder die Gelenkeinrichtung oder ein Gelenkmodul angeordnet sind. Der Adapter kann als separates Bauteil an dem Oberteil und/oder dem Unterteil befestigt sein oder Teil des Oberteil und/oder Unterteils sein, so dass durch die geeignete Wahl der Befestigungseinrichtung, an der der Aktuator und/oder die Gelenkeinrichtung festgelegt wird, der maximal erreichbare Flexions- und/oder Extensionswinkel bestimmt und eingestellt werden kann. Durch die Ausbildung oder Anordnung mehrerer Befestigungseinrichtungen auf einem Adapter ist es möglich, den Aktuator und/oder die Gelenkeinrichtung an unterschiedlichen Positionen auf dem Adapter festzulegen. Jede Position der Befestigungseinrichtung bietet einen anderen Abstand und führt zu einem anderen Extensionsanschlag bei voller Ausnutzung des Extensionsweges für den jeweiligen Aktuator. Die Befestigungsstelle kann an einem auswechselbaren Adapter angeordnet oder ausgebildet sein, an dem ein oder mehrere Befestigungseinrichtungen für den Aktuator und/oder die Gelenkeinrichtung angeordnet oder ausgebildet sind. Statt mehrerer Befestigungseinrichtungen für den Aktuator und/oder der Gelenkeinrichtung kann an dem Adapter auch nur eine Befestigungseinrichtung für den Aktuator und/oder die Gelenkeinrichtung ausgebildet sein, so dass für jeden Extensionsanschlag ein separater Adapter an dem Oberteil und/oder Unterteil angeordnet werden muss. Dies kann zum Ausgleich von Fertigungstoleranzen oder Anpassungen an längerfristige Änderungen in den anatomischen Gegebenheiten des Patienten vorteilhaft sein. Es können verschiedene Adapterplatten mit unterschiedlichen Befestigungseinrichtungen, beispielsweise Lochmustern oder Gewindeeinsätzen für den Aktuator an dem Oberteil und/oder Unterteil befestigt oder angeordnet werden. Die Gelenkeinrichtung kann insbesondere als ein Gelenkmodul ausgebildet sein, das abnehmbar an dem Oberteil und dem Unterteil festlegbar ist. Der Modulaufbau erleichtert die Montage und die Anpassbarkeit an den jeweiligen Nutzer.

In einer Weiterbildung der Erfindung ist es vorgesehen, dass das Oberteil und/oder das Unterteil und/oder zumindest eine kraftübertragende Schnittstelle einer Gelenkeinrichtung längenveränderlich ausgebildet sind. Das Oberteil und/oder das Unterteil und/oder die kraftübertragende Schnittstelle können mehrteilig ausgebildet sein, wobei in einem ersten Teil des Oberteils und/oder Unterteils bzw. der Schnittstelle die Gelenkeinrichtung ausgebildet ist und an einem zweiten Teil die Befestigungsstelle angeordnet oder ausgebildet ist. Das erste Teil und das zweite Teil des Oberteils und/oder des Unterteils bzw. der Schnittstelle sind verlagerbar aneinander befestigt, beispielsweise über einen Rahmen oder eine Schiene, die längenveränderlich ausgebildet ist, so dass die Befestigungsstelle von der Gelenkeinrichtung oder der Gelenkachse weg verlagert werden kann, um dem Extensionsanschlag zu verstellen. Grundsätzlich ist es auch möglich, dass das erste Teil und das zweite Teil teleskopierbar ausgebildet und ineinander einschiebbar und in der jeweiligen Position arretierbar sind. Ebenso ist es möglich, dass das erste Teil oder Oberteil und das zweite Teil oder Unterteil verschwenkbar aneinander angeordnet sind, um darüber unterschiedliche Abstände von Befestigungsstellen relativ zu der Gelenkachse zu erreichen.

Eine Weiterbildung der Erfindung sieht vor, dass der Aktuator ein längenveränderliches Gehäuse und/oder eine längenveränderliche Kolbenstange aufweist, um den maximalen Verlagerungsweg des Aktuator einzustellen. Das Gehäuse kann über ein Gewinde oder eine teleskopierbare Ausgestaltung der Gehäuseteile längenveränderbar ausgebildet sein. Alternativ oder ergänzend kann über eine längenveränderliche Kolbenstange, beispielsweise über eine Schraubhülse, die motorisch oder manuell einstellbar und arretierbar ausgebildet ist.

In einer Weiterbildung der Erfindung ist es vorgesehen, dass zumindest ein Sensor zur Erfassung der zwischen dem Oberteil und dem Unterteil übertragenden Kraft oder des übertragenden Momentes dem Oberteil, dem Unterteil oder der Gelenkeinrichtung zugeordnet ist. Ebenso kann ein Sensor für die Erfassung eines Gelenkwinkels, der Raumlage des Oberteils und/oder Unterteils vorgesehen oder alternativ oder ergänzend ein Sensor zur Erfassung biometrischer Signale eines Nutzers der orthopädietechnischen Einrichtung, z.B. Orthese zugeordnet werden. Als biometrische Signale können beispielsweise dem Bewegungsablauf zuordenbare myoelektrische Signale der Muskulatur der Gliedmaßen, z.B. der Gliedmaße, an der eine Orthese befestigt ist oder die Kompensationsbewegungen ausführt, verwendet werden, um Muskelaktivitäten des Patienten zu erfassen und daraus zu schließen, ob und inwieweit ein Patient oder Nutzer in der Lage ist, bestimmte Bewegungen auszuführen, welche Muskeln wie stark zur Ausführung der Bewegung kontrahiert werden und inwieweit Muskelkontraktionen und Steuereinrichtungen für einen Aktuator oder der Extensionsanschlag medizinischen Vorgaben entsprechen. Die biometrischen Signale können auch an nicht versorgten Gliedmaßen abgenommen werden, um beispielsweise im Rahmen der Auswertung von Kompensationsbewegungen Rückschlüsse ziehen zu können. Auf der Basis der Sensorsignale oder zumindest unter Berücksichtigung der Sensorsignale kann dann eine Einstellung der jeweiligen, den anatomischen Voraussetzungen oder Erfordernissen entsprechenden Extensionsanschlagspositionen erfolgen.

Die orthopädietechnische Einrichtung ist bevorzugt als steuerbare Einrichtung ausgebildet und mit zumindest einem Sensor versehen oder gekoppelt, der mit einer Steuerungseinrichtung gekoppelt ist, die eine Verstelleinrichtung zur Verstellung eines Extensionsanschlages, der einen maximalen Gelenkwinkel begrenzt, aktiviert oder deaktiviert. Der Extensionsanschlag wird in Abhängigkeit von Sensordaten von einer Ausgangsstellung, in der das Oberteil relativ zu dem Unterteil in einer flektierten Stellung befindlich ist, in eine Endstellung verlagert, in der sich das Oberteil relativ zu dem Unterteil in einer gegenüber der Ausgangsstellung veränderten Stellung befindet. Die Steuerungseinrichtung kann ausgebildet sein, die Sensordaten über einen Zeitraum zu erfassen und mit einem Schwellwert zu vergleichen, wobei eine Verstellung des Extensionsanschlages nur dann erfolgt, wenn der Schwellwert über einen definierten Zeitraum erreicht wird.

Eine Weiterbildung sieht vor, dass der maximale Gelenkwinkel einstellbar und der Extensionsanschlag in Abhängigkeit von dem gemessenen Gelenkwinkel bis zum maximalen Gelenkwinkel verstellbar ist. Die Steuerung kann so eingerichtet sein, dass bei Erreichen des Extensionsanschlages das um die Gelenksachse wirksame Moment und/oder die zwischen dem Oberteil und dem Unterteil wirksame Kraft gemessen wird und bei Überschreiten eines Schwellwertes der Extensionsanschlag in Richtung auf seine Endstellung verlagert wird. Bei Unterschreiten des Schwellwertes wird der Extensionsanschlag in entgegengesetzte Richtung verlagert.

Die Steuerung kann weiterhin eingerichtet sein, Widerstandswerte des Aktuators während der Verschwenkbewegung zu erfassen und mit Sensorwerten bezüglich des Gelenkwinkels und der zwischen dem Oberteil und dem Unterteil herrschenden Kräfte und/oder Momente zu korrelieren und bei einer Abweichung eines Kräfte- und Momentenverlaufs von dem Widerstandsverlaufs eine automatische Verstellung des Extensionsanschlages durchzuführen. Bevorzugt ist der Extensionsanschlag autoadaptiv verstellbar ausgebildet.

Ein Verfahren zur Steuerung einer orthopädietechnischen Einrichtung mit einem Oberteil und einem Unterteil, die über eine Gelenkeinrichtung schwenkbar um eine Gelenkachse miteinander verbunden sind und Befestigungseinrichtungen aufweisen, mit denen die orthopädietechnische Einrichtung, z.B. Orthese oder Prothese an einer Gliedmaße festlegbar ist, mit einem Aktuator, der an dem Oberteil und dem Unterteil an Befestigungsstellen festgelegt ist und eine Verschwenkung des Oberteils relativ zu dem Unterteil beeinflusst, und mit zumindest einem Sensor, der mit einer Steuerungseinrichtung gekoppelt ist, die eine Verstelleinrichtung zur Verstellung eines Extensionsanschlages, der einen maximalen Gelenkwinkel begrenzt aktiviert oder deaktiviert, sieht vor, dass der Extensionsanschlag in Abhängigkeit von Sensordaten von einer Ausgangsstellung, in der das Oberteil relativ zu dem Unterteil in einer flektierten Stellung befindlich ist, in eine Endstellung verlagert wird, in der sich das Oberteil relativ zu dem Unterteil in einer gegenüber der Ausgangsstellung veränderten, insbesondere extendierten Stellung befindet. Der Extensionsanschlag kann neben einer mechanischen Begrenzung auch durch Veränderung eines hydraulischen Systems limitiert oder verändert werden, zum Beispiel durch das gesteuerte Öffnen und Schließen von Ventilen oder Sperreinrichtungen, die eine Weiterverlagerung eines Kolbens innerhalb eines Zylinders bei einer vorbestimmten, jedoch auf der Grundlage von Sensordaten veränderbaren Stellung verhindern.

Der maximal mögliche Gelenkwinkel ist somit nicht fixiert, sondern kann verstellt werden. Als Ausgangsstellung ist zunächst ein maximaler Gelenkwinkel eingestellt, der flektiert ist, also eine Einbeugung beispielsweise des Orthesenkniegelenkes vorsieht. Der maximale Gelenkwinkel liegt unterhalb der maximal extendierten Stellung von 180°, also einer senkrechten Orientierung der Längserstreckung des Oberteils relativ zu dem Unterteil. Grundsätzlich ist auch eine Hyperextension, also eine Überstreckung als maximaler Gelenkwinkel möglich. Bei einem Orthesenkniegelenk liegt eine Hyperextension dann vor, wenn die Rückseite des Unterschenkels relativ zu der Rückseite des Oberschenkel im einem Winkel von größer 180° steht, eine Hyperextension des Ellbogengelenkes liegt vor, wenn der Unterarm von der Bizepsseite des Oberarms über einen Winkel über 180° gestreckt wird oder aber der Unterarm auf der Trizepsseite des Oberarms einen Winkel von kleiner 180° einschließt. Durch dieses Verfahren wird beispielsweise eine Änderung der physiologischen Gegebenheiten bei einem Patienten über den Verlauf des Tragens der Orthese Rechnung getragen. Während morgens aufgrund einer Beugekontraktion der Bewegungsumfang verringert sein kann, also der von dem Patienten ausführbare maximale Gelenkwinkel gegenüber einer maximalen Extension in einer flektierten Stellung befindlich ist, kann sich durch die Bewegung während des Tages die Beugekontraktion verringern. Würde der Extensionsanschlag statisch bleiben, würde entweder morgens ein zu großer Extensionsanschlag eingestellt werden, was dazu führen würde, dass die Gewebestrukturen des Patienten überbeansprucht werden würden. Zusätzlich kann es zu Problemen bei der Steuerung von Orthesen führen, bei denen der Extensionsanschlag als ein Auslösesignal verwendet wird. Würde der Extensionsanschlag auf dem Niveau der morgendlichen Beugekontraktion eingestellt bleiben, würde dies zwar eine dauerhafte Signalauslösung durch Erreichen des Extensionsanschlages bei jeder vollständigen Bewegung bedeuten, allerdings würde dies die Beugekontraktion verfestigen und darüber hinaus einen Komfortverlust bedeuten.

Wird auf Grundlage von Sensordaten jedoch eine automatische Anpassung des Extensionsanschlages durchgeführt, können einerseits Veränderungen in dem Bewegungsumfang während eines Tages berücksichtigt werden und andererseits Veränderungen oder Fortschritte bei der Genesung, insbesondere bei der Erhöhung des Bewegungsumfanges berücksichtigt werden. So kann über eine lang andauernde Betrachtung von Sensorwerten wie Gelenkwinkel, Kräfte, Momente, Beschleunigungen, Raumlagen oder auch myoelektrischer oder anderer physiologischer Signale erfasst werden, ob der Nutzer Fortschritte hinsichtlich der Mobilität und des jeweiligen Bewegungsumfanges erzielt hat, so dass über einen langen Zeitraum hinweg, beispielsweise mehrere Monate, der maximale Extensionsanschlag bis zu derjenigen Grenze verlagert wird, bei der die anatomisch maximale Extension erreicht ist. Zusätzlich können die Informationen, die durch den Sensor oder die Sensoren bereitgestellt werden, über einen vorgegebenen Zeitraum gespeichert und ausgewertet werden, um zu Dokumentationszwecken verwendet werden zu können. Damit lassen sich zum Beispiel Therapiefortschritte dokumentieren.

Die Sensordaten können über einen Zeitraum erfasst und mit einem Schwellwert verglichen werden, wobei die Verstellung des Extensionsanschlages nur dann erfolgt, wenn der Schwellwert über einen definierten Zeitraum erreicht wird. Statt nur einer momentanen Messung wird mit dem Sensor oder werden mit dem Sensor eine Vielzahl von Daten oder auch Datenverläufen erfasst und mit einem Schwellwert verglichen, der über einen gewissen definierten Zeitraum erreicht werden muss. Erst wenn ein Mobilitätsniveau oder Extensionsniveau über einen gewissen Zeitraum erreicht wird, wird eine Verstellung des Extensionsanschlages in Richtung einer zunehmenden Extension durchgeführt, um zu verhindern, dass es sich bei der erfassten und zu bewertenden Situation nicht um einen statistischen Ausreißer, um eine untypische Bewegung oder um eine Fehlmessung handelt.

Der maximale Gelenkwinkel kann eingestellt werden und wird so eingestellt, dass sich der Bewegungsumfang der Orthese innerhalb jener Grenzen befindet, die medizinisch sinnvoll sind und sich innerhalb der anatomischen Gegebenheiten des jeweiligen Patienten befinden. Der Extensionsanschlag kann dann in Abhängigkeit von dem jeweils gemessenen Gelenkwinkel bis hin zum maximal erreichbaren Gelenkwinkel oder zum Therapieziel verstellt werden. Der jeweils gemessene Gelenkwinkel wird mit einem Sollwert, Schwellwert oder einem Grenzwert verglichen. Der Sollwert, Schwellwert oder Grenzwert kann über einen längeren Zeitraum langsam angepasst werden, beispielsweise durch eine laufende Mittelwertbildung des gemessenen Winkels. Die Veränderung des Extensionsanschlages endet, wenn der vordefinierte maximale Gelenkwinkel als definierter Grenzwert erreicht wird, um eine Überdehnung der anatomischen Strukturen zu verhindern. Die Einstellung des Extensionsanschlages kann auch auf der Grundlage dessen erfolgen, dass zu einem vorgegebenen Zeitpunkt das übertragene Gelenksmoment oder die übertragenen Kräfte bewertet werden. So kann beispielsweise festgelegt werden, dass bei einer Orthese beim Gehen in der Ebene in der gestreckten Position des Kniegelenkes ein bestimmtes Moment von der Orthese übertragen werden muss. Ist das tatsächlich übertragene Moment zu gering, deutet dies auf eine mögliche Überlastung der anatomischen Strukturen hin, woraus geschlossen werden kann, dass der Streckanschlag oder Extensionsanschlag der Orthese verstellt werden musste, um bei einem geringeren Gelenkwinkel bereits in den Anschlag zu gehen. Ist das übertragene Moment sehr hoch, kann dies ein Indikator dafür sein, dass die Orthese zu früh an den Streckanschlag anschlägt, das Kniegelenk also noch weiter in die Extension gehen könnte und die Orthese die Bewegung zu früh sperrt. Ein gewünschter Dehnungseffekt für den Patienten wird somit nicht erreicht, die Bewegung kann nicht vollständig und korrekt ausgeführt werden, wodurch das Orthesensystem insgesamt ineffizient werden würde. Werden also sehr hohe Kräfte oder Momente bei Erreichen des Extensionsanschlages in der Orthese festgestellt, kann dies zur Verlagerung des Extensionsanschlages in einer Richtung zu einem vergrößerten Gelenkwinkel führen.

Alternativ oder ergänzend wird bei der Verstellung des Extensionsanschlages berücksichtigt, ob und inwieweit bei Erreichen des Extensionsanschlages in definierten Bewegungsphasen ein Schwellwert für das um die Gelenkachse wirksame Moment oder der zwischen dem Oberteil und dem Unterteil wirksamen Kräfte erreicht oder überschritten wird. Dazu werden bei Erreichen des momentanen Extensionsanschlages das um die Gelenkachse wirksame Moment oder die zwischen dem Oberteil und dem Unterteil wirksamen Kräfte gemessen und bei Überschreiten eines Schwellwertes der Extensionsanschlag in Richtung auf seine Endstellung verlagert. Bei Unterschreiten des Schwellwertes kann der Extensionsanschlag in die entgegengesetzte Richtung verlagert werden, also der maximal erreichbare Gelenkwinkle verringert werden, beispielsweise um sicherzustellen, dass bei jeder vollständigen Bewegung der Extensionsanschlag erreicht wird, ohne die anatomischen Strukturen zu überdehnen oder sehr zu belasten. Die Festlegung der definierten Bewegungsphasen verhindert eine ungewollte Verstellung bei zufällig auftretenden hohen Momenten, beispielsweise beim Stolpern.

Weiterhin ist es möglich, dass Widerstandswerte des Aktuators während der Verschwenkbewegung erfasst und mit Sensorwerten bezüglich des Gelenkwinkels und der zwischen dem Oberteil und dem Unterteil herrschenden Kräfte und/oder Momente korreliert werden. Die Adaption oder Verstellung des Extensionsanschlages erfolgt somit aufgrund eines Zusammenhangs zwischen Gelenkwinkeln, Momenten oder Kräfteverläufen sowie Widerstandswerten. Anatomische Gelenke weisen im Allgemeinen keinen harten Streckanschlag auf, sondern zeigen eine gewissen Elastizität aufgrund der Bänder, Sehnen und Kapseln rund um den Streckanschlag. Diese Elastizität führt bei gewissen Bewegungen wie beispielsweise in der Schwungphasenextension oder Standphasenextension beim Gehen zu einem charakteristischen Winkel-Momenten-Verlauf. Dabei wird die Streckbewegung über jenes Maß hinaus abgebremst, das von dem Hilfsmittel selbst beeinflusst wird. Der Winkelbereich und die Momente werden ermittelt und mit einem charakteristischen Winkel-Momenten-Verlauf verglichen. Wird der charakteristische Winkel-Momenten-Verlauf nicht erreicht, ist dies ein Indikator dafür, dass der Extensionsanschlag angepasst werden muss. So kann beispielsweise für das Kniegelenk in der Schwungphasenextension beim Gehen ermittelt werden, ob das Gelenk in einem Winkelbereich vor dem Endanschlag stark abgebremst wird, obwohl die Orthese mit dem Aktuator einen konstant geringen Widerstand bietet. Damit kann geschätzt werden, wo der natürliche Endanschlag liegt. Die Position des Extensionsanschlages kann dann automatisch so angepasst werden, dass dieser Effekt kurz vor dem Endanschlag auftritt, um einen möglichst natürlichen Bewegungsablauf zu erreichen.

Bevorzugt wird der Extensionsanschlag autoadaptiv verstellt, also auf Grundlage von gemessenen Sensorwerten ohne Eingriff eines Patienten oder Orthopädietechnikers aufgrund vorher festgelegter Kriterien. Dazu werden von den Sensoren Informationen erfasst, die die Grundlage zu der Beurteilung einer möglichen Änderung der Ortheseneigenschaft, insbesondere des Extensionsanschlages bilden. Neben von Sensoren erfassten Messwerten können auch berechnete oder geschätzte Größen und Zustände des Systems, also der Orthese zusammen mit dem Patienten verwendet werden, diese können sowohl die Größen und Zustände über einen zurückliegenden Zeitraum als auch aktuelle Größen und Zustände sein. Neben Winkeln, Momenten, Kräften oder Positionen von Oberteil und Unterteil im Raum oder auch von Gliedmaßen im Raum, sind insbesondere Informationen hinsichtlich der Tagesdauer, der Tageszeit oder des Verlaufs der bisherigen Anpassung, beispielsweise in welchen Zeiträumen welche Veränderungen des Extensionsanschlages in Richtung eines zunehmenden maximalen Gelenkwinkels oder eines abnehmenden maximalen Gelenkwinkels erreicht worden sind. Insofern kann eine Auswertung von elektromyographischen Sensoren zweckmäßig sein, um Aufschluss der Aktivitäten der Restmuskulatur und gegebenenfalls vorhandener Spastiken zu bekommen.

In einer Steuerungseinrichtung mit einer Recheneinheit, in der Sensoren Daten und andere Informationen verarbeitet und gegebenenfalls gespeichert sind, werden die vorhandenen Informationen verarbeitet, miteinander verglichen und Korrelationen ermittelt, beispielsweise die Ermittlung des Gelenkmoments aus einem Gelenkwinkel und einer Hydraulikkraft in dem Aktuator unter Berücksichtigung der vorhandenen geometrischen Bedingungen in der Orthese. Diese Informationen werden zu einem oder mehreren Kriterien zusammengefasst und jeweils der Bewertung zugrundeliegende Beobachtungszeitraum kann je nach Anwendungszweck variieren. Darüber hinaus können für unterschiedliche Informationen unterschiedliche Beobachtungszeiträume herangezogen werden, beispielsweise Sensordaten des aktuellen Gangzyklusses und eine Anpassung des Extensionsanschlages über die letzten hundert oder mehr Schritte. Daraus können Kriterien wie Extremwerte, Mittelwerte, Differenzen, integrale Standardabweichung oder auch komplexere Merkmale wie Datenreihen, Typographien, Korrelationen abgeleitet werden. Anschließend werden die Kriterien bewertet oder mit Referenzgrößen verglichen, die unter anderem auch auf patientenspezifischen Daten beruhen können. Die Bewertung kann mit Klassifizierungsmethoden wie neuronalen Netzen, Machine Learning oder Mustererkennung erfolgen. In einer Bewertungseinrichtung wird auf Basis der aus den Sensordaten ermittelten Kenngrößen entschieden, ob und inwieweit eine Veränderung des Extensionsanschlages notwendig ist und wie groß diese Veränderung sein soll.

Der Extensionsanschlag kann zu der Durchführung des Verfahrens durch eine Verlagerung der Befestigungsstellen oder durch jede andere Veränderung erreicht werden, z.B. durch eine gesteuerte Sperrung von Hydraulikleitungen, durch eine Veränderung der Länge der Kolbenstange, durch Einstellen von mechanischen Endanschlägen oder durch andere Veränderungen an der Gelenkeinrichtung, dem Oberteil, dem Unterteil und/oder dem Aktuator.

Nachfolgend werden Ausführungsbeispiele der Erfindung anhand der beigefügten Figuren näher erläutert. Es zeigen:
- Figur 1 -: eine schematische Darstellung einer orthopädietechnischen Einrichtung in Seitenansicht;
- Figur 2 -: eine Variante einer orthopädietechnischen Einrichtung in Frontalansicht;
- Figur 3 -: eine Detailansicht einer Gelenkeinrichtung;
- Figur 4 -: eine Variante der Figur 3;
- Figur 5 -: eine Detailansicht einer Verstelleinrichtung zur verschieblichen Verstellung einer Befestigungsstelle;
- Figur 6 -: eine Detaildarstellung einer verdrehbaren Befestigungsstelle;
- Figur 7 -: Darstellungen unterschiedlicher Adapter;
- Figur 8 -: unterschiedliche Adapterplatten für die orthopädietechnische Einrichtung;
- Figur 9 -: eine Variante der Erfindung mit einem längenveränderlichen Oberteil;
- Figur 10 -: eine Variante mit einer längenveränderlichen Kolbenstange;
- Figur 11 -: eine Detailschnittdarstellung einer längenveränderlichen Kolbenstange;
- Figur 12 -: eine weitere Variante mit verschiedenen Verstellmöglichkeiten; sowie
- Figur 13 -: eine alternative Möglichkeit zur einstellbaren Orientierung eines Unterteils relativ zu an einem Oberteil festlegbaren Gliedmaße.

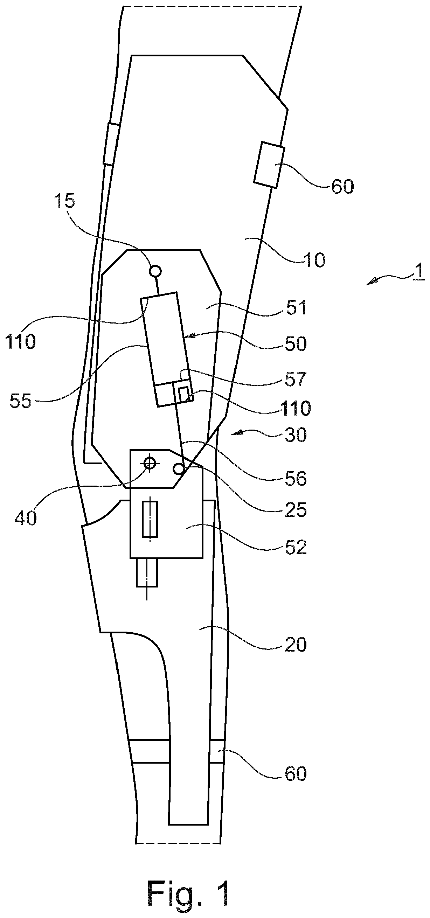

In der Figur 1 ist in einer schematischen Darstellung in Seitenansicht eine orthopädietechnische Einrichtung 1 in einer gelenkübergreifenden Orthese dargestellt. Die orthopädietechnische Einrichtung 1 weist ein Oberteil 10 und ein Unterteil 20 auf, die über eine Gelenkeinrichtung 30 schwenkbar um eine Gelenkachse 40 miteinander verbunden sind. An dem Oberteil 10, das als eine Schiene oder Schale ausgebildet sein kann, ist eine Befestigungseinrichtung 60 in Gestalt eines Gurtes angeordnet, um das Oberteil 10 an einer Gliedmaße, im dargestellten Ausführungsbeispiel an einem Oberschenkel, festzulegen. Korrespondierend ist an dem Unterteil 20 eine Befestigungseinrichtung 60 angeordnet, um das Unterteil 20 mit dem Unterschenkel zu koppeln und daran festzulegen. Bei einer Ausgestaltung der orthopädietechnischen Einrichtung 1 als eine Armorthese wird das Oberteil 10 an einem Oberarm und das Unterteil 20 an einem Unterarm festgelegt. Bei einer Ausgestaltung der orthopädietechnischen Einrichtung als eine Prothese ist das Oberteil 10 als ein Prothesenschaft ausgebildet, in dem beispielsweise über einen Liner und eine Unterdruckquelle die Gliedmaße festgelegt wird. In dem dargestellten Ausführungsbeispiel ist die Gelenkeinrichtung 30 einseitig, vorzugsweise lateral an der Gliedmaße angeordnet, alternative Ausgestaltungen mit zwei Gelenkeinrichtungen 30, eine medial und eine lateral an der Gliedmaße angeordnet, sind ebenfalls vorgesehen. Statt einer monozentrischen Ausgestaltung der Gelenkeinrichtung 30 mit nur einer Gelenkachse 60 kann die Gelenkeinrichtung 30 auch ein Mehrachsengelenk ausbilden, was sowohl bei Prothesen als auch Orthesen sinnvoll ist.

An dem Oberteil 10 und dem Unterteil 20 ist ein Aktuator 50 an Befestigungsstellen 15, 25 festgelegt. Die Festlegung erfolgt in dem dargestellten Ausführungsbeispiel über kraftübertragende Schnittstellen 51, 52, die als Trägerplatten, Teile des Oberteils 10 oder Unterteils 20, Adapterplatten, Rahmenkonstruktionen oder ähnliches ausgebildet sein können. Auch eine unmittelbare Befestigung des Aktuators 50 an dem Oberteil 10 und dem Unterteil 20 ohne Zwischenschaltung kraftübertragender Schnittstellen 51, 52 ist vorgesehen und wird später erläutert. Der Aktuator 50 gemäß Figur 1 ist ein Hydraulikdämpfer, der ein Gehäuse 55 aufweist, in dem längsbeweglich ein Hydraulikkolben 57 angeordnet ist, der über eine Kolbenstange 56, die aus einem Zylinder innerhalb des Gehäuses 55 hinausragt, mit dem Unterteil 20 an der Befestigungsstelle 25 gekoppelt ist. Das Gehäuse 55 ist an dem Oberteil 10 über die kraftübertragende Schnittstelle 51 an einer daran angeordneten oder ausgebildeten Lageraufnahme festgelegt. Die Befestigungsstelle 25 an der kraftübertragenden Schnittstelle 52 zur Festlegung an dem Unterteil 20 befindet sich beabstandet von der Gelenkachse 40, so dass bei einer Verschwenkung des Unterteils 20 um die Gelenkachse 40 die Kolbenstange 56 zusammen mit dem Kolben 57 innerhalb des Gehäuses 55 des Aktuators 50 bewegt wird. In der dargestellten, gestreckten, also maximal extendierten Stellung der orthopädietechnischen Einrichtung 1 ist die Kolbenstange 56 maximal ausgefahren, der Kolben 57 liegt an einem Extensionsanschlag 110 an, eine weitere Schwenkung in Extensionsrichtung, also zur Vergrößerung des Gelenkwinkels zwischen dem Oberteil 10 und dem Unterteil 20 auf der Rückseite oder Beinbizepsseite der orthopädietechnischen Einrichtung 1 ist nicht möglich. Wird das Unterteil 20 entgegen dem Uhrzeigersinn um die Gelenkachse 40 verschwenkt, wird eine Flexionsbewegung eingeleitet, wodurch der Kolben 57 innerhalb des Gehäuses 55 nach oben in Richtung auf die proximale Befestigungsstelle 15 verlagert wird. Der Extensionsanschlag 110 kann einstellbar sein, beispielsweise indem dessen Position durch Hineinschrauben oder Herausschrauben aus dem Gehäuse 50 verändert wird. Alternativ zu einer Anordnung eines Gelenkanschlages, insbesondere Extensionsanschlages 110 in dem Aktuator kann ein entsprechender Extensionsanschlag 110 auch in der Gelenkeinrichtung 30 angeordnet sein, um die Orientierung des Oberteils 10 und des an dem Oberteil 10 festgelegten Oberschenkels relativ zu dem Unterschenkel oder dem Unterteil 20 einzustellen. Im Rahmen einer Therapie kann es notwendig sein, den Extensionsanschlag zu verändern, beispielsweise um Schädigungen an einem Bandapparat oder an Geweben zu vermeiden oder um im Rahmen eines Therapiefortschrittes oder einer Heilung eine vergrößerte Streckbewegung oder Extension durchführen lassen zu können.

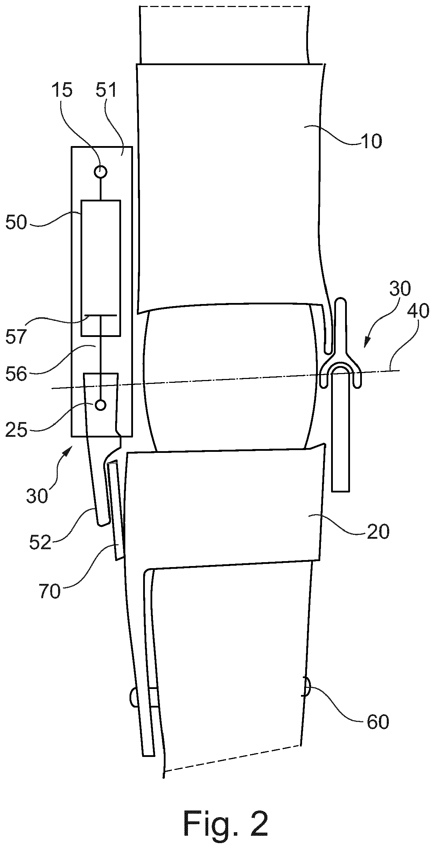

Figur 2 zeigt eine Variante einer orthopädietechnischen Einrichtung 1 in Gestalt einer kniegelenksübergreifenden Orthese mit einem manschettenartigen Oberteil 10, einem Unterteil 20 und einer Gelenkeinrichtung 30, die durch die kraftübertragenden Schnittstellen 51, 52 ausgebildet ist. Der Aktuator 50 ist beispielsweise lateral an dem Bein angeordnet, medial an dem Oberteil 10 und dem Unterteil 20 ist eine zweite Gelenkeinrichtung 30 angeordnet, die als ein sogenanntes Mitläufergelenk ausgebildet ist und durch das ebenfalls die Gelenkachse 40 hindurchläuft. Das Oberteil 10 und das Unterteil 20 sind über die Befestigungseinrichtungen 60 an dem Oberschenkel bzw. Unterschenkel angeordnet.

Alternativ oder ergänzend zu einem Extensionsanschlag 110 in dem Aktuator 50, wie es in der Figur 1 dargestellt ist, kann an einer der Gelenkeinrichtungen 30 oder an beiden Gelenkeinrichtungen 30 ein verstellbarer Anschlag zur Begrenzung des Flexionswinkels und/oder der Extensionswinkels angeordnet sein.

In der Figur 2 ist weiterhin zu erkennen, dass zwischen der distalen kraftübertragenden Schnittstelle 52 und dem Unterteil 20 ein Adapter 70 in Gestalt einer Adapterplatte angeordnet ist. Über die Adapterplatte ist es möglich, einen Winkelausgleich bereitzustellen, beispielsweise um Fertigungstoleranzen oder Abweichungen in den anatomischen Gegebenheiten unterschiedlicher Patienten auszugleichen. Ebenso ist es möglich, über den Adapter 70 einen Abstandsausgleich herbeizuführen, um mediale und/oder laterale Abstände oder Lücken zwischen dem Unterteil 20 und der kraftübertragenden Schnittstelle 52 auszugleichen. Dies kann beispielsweise durch die unterschiedlich dicke Adapter- oder Adapterplatten 70 erreicht werden. An dem jeweiligen Adapter 70 kann eine Befestigungsstelle angeordnet oder ausgebildet sein. Ebenso ist es möglich, mehrere Lageraufnahmen oder Ähnliches daran vorzusehen, um entweder, wie in dem dargestellten Ausführungsbeispiel, die Gelenkeinrichtung 30, die als Gelenkmodul ausgebildet ist, daran an unterschiedlichen Stellen oder Positionen zu befestigen oder aber, um den Aktuator 50 ohne Zwischenschaltung von kraftübertragenden Schnittstellen 51, 52 an dem jeweiligen Oberteil 10 oder Unterteil 20 festlegen zu können. Dazu sind an dem Adapter 70 geeignete Einrichtungen angeordnet oder ausgebildet, um die jeweilige Komponente oder das jeweilige Bauteil daran festzulegen. Ergänzend zu der Anordnung eines Adapters 70 an dem Unterteil 20 kann ein solcher Adapter 70 oder ein Adapter 70 einer anderen Bauart auch an dem Oberteil 10 angeordnet sein, um unterschiedliche Befestigungsmöglichkeiten der kraftübertragenden Schnittstelle 51 oder direkt des Aktuators 50 bereitzustellen. Ein Adapter 70 kann sowohl an dem Oberteil 10 als auch an dem Unterteil 20 oder nur an einer dieser Komponenten angeordnet sein.

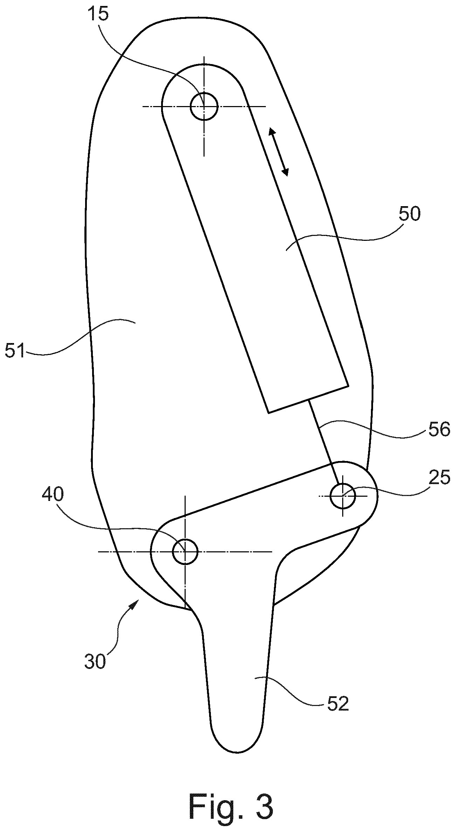

In der Figur 3 ist eine Detailansicht der Gelenkeinrichtung 30 mit den kraftübertragenden Schnittstellen 51, 52 mit der gelenkigen Verbindung um die Gelenkachse 40 dargestellt. Die unterteilseitige Schnittstelle 52 ist als Winkel ausgebildet, die oberteilseitige Schnittstelle 51 als eine Platte. Grundsätzlich ist es auch möglich, den Aktuator 50 unmittelbar an einem Oberteil und Unterteil anzuordnen oder aber direkt an einem Oberteil oder Unterteil und über eine Schnittstelle 51, 52 an der entsprechend andere Orthesenkomponente. Um den maximalen Extensionswinkel und gegebenenfalls den maximalen Flexionswinkel zwischen den beiden Schnittstellen 51, 52 und damit auch zwischen dem Oberteil 10 und dem Unterteil 20 verändern zu können, ist in dem dargestellten Ausführungsbeispiel die obere Befestigungsstelle 15 verschieblich an der kraftübertragenden Schnittstelle 51 gelagert, so dass der Abstand zwischen der Befestigungsstelle 15 und der Gelenkachse 40 verändert wird. Bei der gleichbleibenden Konfiguration innerhalb des Aktuators 50, also bei einem mechanischen Endanschlag des Kolbens innerhalb des Zylinders, verändert sich dadurch die maximal erreichbare Winkelstellung zwischen dem Oberteil 10 und dem Unterteil 20. Wird beispielsweise die Befestigungsstelle 15 nach oben, von der Gelenkachse 40 weg verlagert, flektiert das Unterteil und der Extensionsanschlag, beispielsweise wenn der Hydraulikkolben an dem unteren, distalen Ende des Zylinders anschlägt, wird nicht bei einer maximal extendierten Stellung erreicht, sondern einer leicht flektierten Stellung. Wird hingegen die Befestigungsstelle 15 in Richtung auf die Gelenkachse 40 verlagert, kann das Unterteil 20 relativ zu dem Oberteil 10 weiter extendieren, bis eine gewünschte oder medizinisch vertretbare Endposition der orthopädietechnischen Einrichtung erreicht ist.

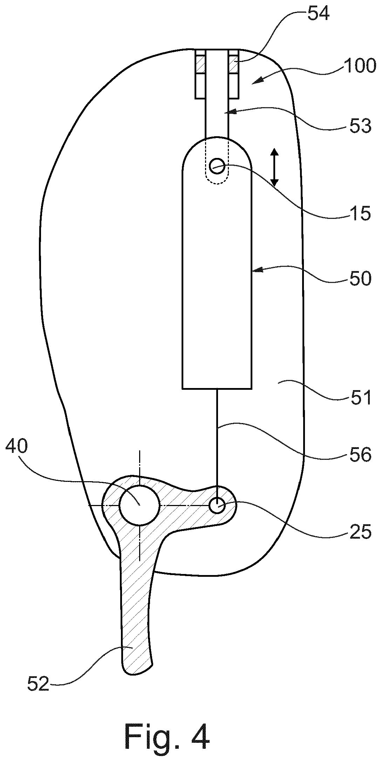

Figur 4 zeigt eine Variante der Verstellung, bei der der Aktuator 50 an einer Aktuatoraufhängung 53 befestigt ist. Zwischen der Aktuatoraufhängung 53 und dem Aktuator 50 ist die Befestigungsstelle 15 ausgebildet. Die Aktuatoraufhängung 53 weist an ihrem oberen Ende ein Außengewinde auf, das mit einem drehbar gelagerten, axial gesicherten Innengewinde 54, das beispielsweise in einer verdrehbaren Hülse ausgebildet ist, in eingriff steht. Die Hülse kann manuell oder motorisch in die eine oder andere Richtung verdreht werden und bildet eine Verstelleinrichtung 100 aus, über die, je nach Drehrichtung der Hülse mit dem Innengewinde 54, die Aktuatoraufhängung 53 und damit der gesamte Aktuator 50 nach oben oder unten verlagert wird, also von der Gelenkachse 40 weg oder in Richtung auf die Gelenkachse 40 hin. Wird der Aktuator 50 zusammen mit der Aktuatoraufhängung 53 nach unten bewegt, kann die kraftübertragende Schnittstelle 52, die an dem Unterteil 20 festlegbar ist, weiter im Uhrzeigersinn um die Gelenkachse 40 verschwenkt werden, wodurch die Orientierung des Oberteils relativ zu dem Unterteil hinsichtlich der maximalen Extensionsstellung verändert werden kann. Wird die Aktuatoraufhängung 53 nach oben von der Gelenkachse 40 weg verlagert, wird die maximal extendierte Stellung bereits erreicht, wenn die orthopädietechnische Einrichtung noch eine flektierte Stellung einnimmt, also das Unterteil 20 relativ zu dem Oberteil 10 einen Winkel einnimmt, der kleiner als 180° zwischen den Längserstreckungen des Oberteils und des Unterteils ist. Zur Verstellung des Extensionsanschlages ist dabei keinerlei Änderung oder Modifikation des Aktuators 50, der als Dämpfer oder auch als Antrieb ausgebildet sein kann, notwendig, es können Standardaktuatoren 50 eingesetzt werden, die in ihrer Position an dem Oberteil 10 oder Unterteil 20 oder den jeweiligen kraftübertragenden Schnittstellen 51, 52 veränderlich festlegbar sind.

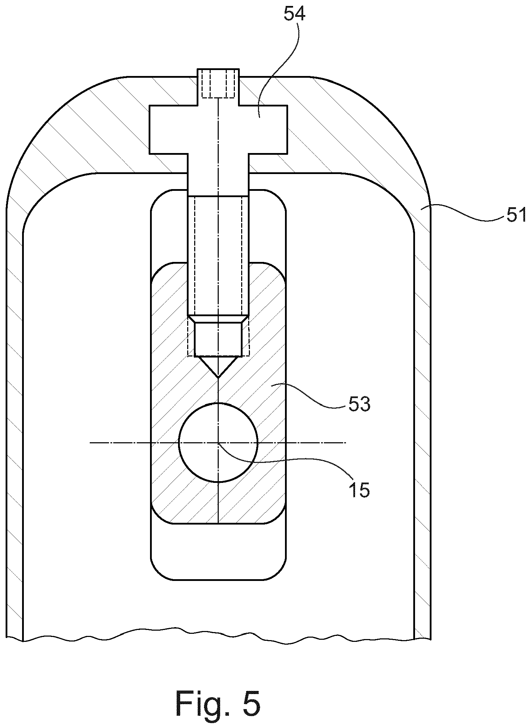

Figur 5 zeigt eine Detailansicht einer möglichen Verstellvorrichtung 100, bei der nur ein Teil der kraftübertragenden Schnittstelle 51 gezeigt ist. Die Befestigungsstelle 15 ist an einem Schiebeelement 53 angeordnet, das längsverschieblich in einer Ausnehmung in der kraftübertragenden Schnittstelle 51 oder in einem Oberteil 10 oder Unterteil 20 angeordnet ist. Über eine Stellschraube 54, die axial unbeweglich und verdrehbar an oder in der kraftübertragenden Schnittstelle 51 gelagert ist, kann das Schiebelement 53 verschoben werden. Durch manuelles oder motorisches Verdrehen der Stellschraube 54 wird die Befestigungsstelle 15 entlang der Ausnehmung in die eine oder andere Richtung verschoben, wodurch der gesamte Aktuator 50 verlagert und damit die Orientierung eines Unterteils relativ zu der an dem Oberteil festlegbaren Gliedmaße einstellbar ist. Insbesondere werden der maximale Flexionswinkel und der maximale Extensionswinkel zwischen dem Oberteil und dem Unterteil eingestellt.

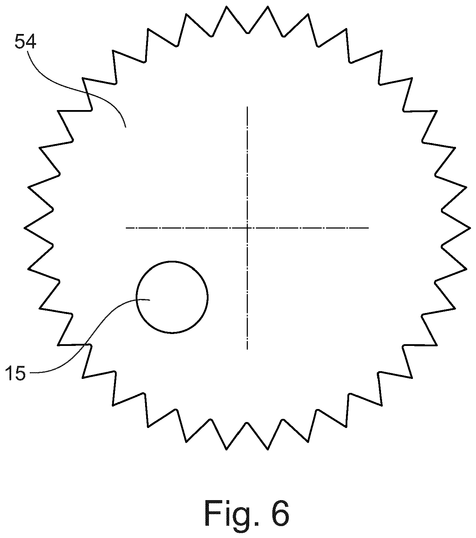

Eine alternative Ausgestaltung einer Verstelleinrichtung 100 ist in der Figur 6 in einer Detaildarstellung gezeigt, bei der die Befestigungsstelle 15 oder 25, beispielsweise ein Zapfen, eine Schraube, ein Gewindeeinsatz oder eine Ausnehmung, exzentrisch zu einer Drehachse eines verzahnten Exzenters 54 angeordnet ist. In die Zähne des verzahnten Exzenters 54 kann eine Lasche oder eine korrespondierende Verzahnung einer Verriegelungseinrichtung eingreifen, um den verzahnten Exzenter54 gegenüber einer ungewollten Verdrehung zu sichern. Durch die exzentrische Anordnung der Befestigungsstelle 15 auf dem verzahnten Exzenter54 ist es möglich, durch Verdrehen eine Verlagerung der Befestigungsstelle 15 relativ zu der Gelenkachse und somit eine Verstellung des Extensionsanschlages und ebenfalls des Flexionsanschlages der orthopädietechnischen Einrichtung zu bewirken. Der verzahnte Exzenter 54 kann manuell oder motorisch verstellt werden, eine Rastlasche zur Verdrehsicherung kann federbelastet in Richtung auf die Außenverzahnung des verzahnten Exzenters54 ausgebildet sein. Die Verzahnung des Exzenters kann auch als eine Verdrehsicherung genutzt werden, wenn sich eine entsprechende Gegenkontur an der kraftübertragenden Schnittstelle 51, 52 bzw. dem Oberteil 10 oder dem Unterteil 20 befindet. In diesem Fall sind anstelle einer Verzahnung auch andere Konturen als Verdrehsicherung denkbar und vorgesehen.

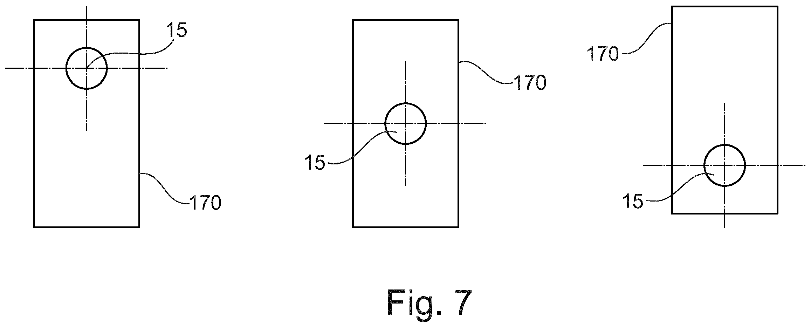

Figur 7 zeigt eine Variante der Erfindung, bei der statt einer verschieblichen Lagerung gemäß Figur 5 eine auswechselbare Komponente 170 als Einleger oder dergleichen in einer Ausnehmung in dem Oberteil 10, dem Unterteil 20 oder einer der beiden Schnittstellen 51, 52 eingelegt werden kann. An der jeweiligen auswechselbaren Komponente 170 ist eine Befestigungsstelle 15 zum Festlegen und Montieren des Aktuators 50 angeordnet oder ausgebildet. Wird der linke Einleger oder die auswechselbare Komponente 170 in die Ausnehmung beispielsweise gemäß Figur 5 eingesetzt, ist diese Befestigungsstelle 15 maximal zu der Gelenkachse 40 entfernt. Eine Begrenzung der Extensionsbewegung wird vor Erreichen des maximalen Gelenkwinkels, der in der Regel bei einer maximalen Streckung der Gliedmaße erreicht ist, erreicht, so dass ein Endanschlag bei einer flektierten Stellung gegeben ist. In der mittleren Darstellung ist die auswechselbare Komponente 170 mit einer mittig angeordneten Befestigungsstelle 15 ausgestattet, so dass dessen Verwendung der Extensionsschlag weiter nach vorne verlagert wird, eine Sperrung der Extensionsbewegung somit später erfolgt. Die rechte Darstellung zeigt die auswechselbare Komponente 70 mit der Befestigungsstelle 15 in der tiefsten Position, in der das Unterteil 20 und das Oberteil 10 in einer maximal gestreckten Stellung befindlich sind, wenn der Aktuator 50 seinen jeweils konstruktiv vorgegebenen Endanschlag erreicht. Die Verschiebung der Befestigungsstelle 15 kann sowohl in proximal-distal-Richtung als auch in anterior-posterior-Richtung erfolgen. Alternativ ist jede andere Außenkontur der auswechselbaren Komponente 170 denkbar, solange sie eine ausreichende Verdrehsicherung sicherstellt.

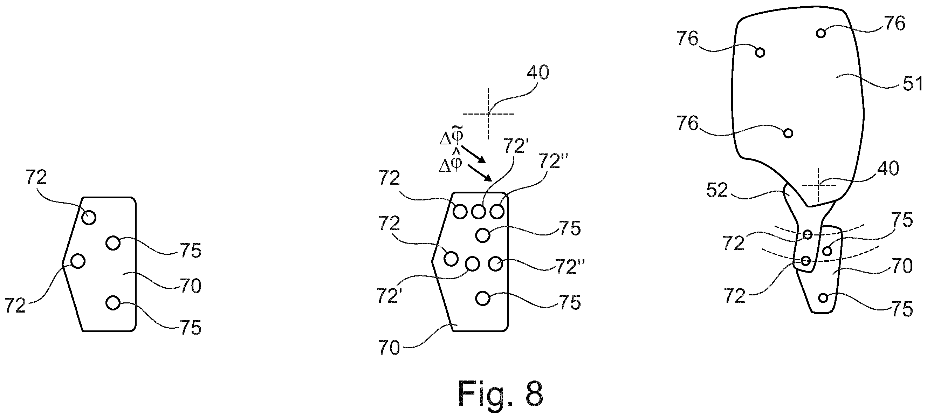

Figur 8 zeigt eine weitere Variante der Erfindung, bei der unterschiedliche Adapter 70 in den beiden linken Darstellungen gezeigt sind. In der rechten Darstellung ist der Adapter 70 an der kraftübertragenden Schnittstelle 52 zur Befestigung an dem nicht dargestellten Unterteil 20 angeordnet. An dem Adapter 70 sind zwei Befestigungseinrichtungen 75 angeordnet oder ausgebildet, an denen zum Beispiel die kraftübertragenden Schnittstelle 52 oder der Aktuator 50, insbesondere die Kolbenstange 57 festgelegt werden kann. Der Adapter 70 weist zwei Ausnehmungen oder Befestigungselemente 72 auf, mit denen der Adapter 70 an der kraftübertragenden Schnittstelle 52 festlegbar ist. In der linken Darstellung der Figur 8 ist der Adapter 70 in einer Einzeldarstellung gezeigt, aus der zu entnehmen ist, dass die beiden Befestigungseinrichtungen 75 beabstandet zueinander ausgebildet sind.

Alternativ können an dem Oberteil 10 oder an der kraftübertragenden Schnittstelle 51 an unterschiedlichen Stellen Befestigungseinrichtungen 76 angeordnet sein, an denen entweder der Aktuator 50 oder die Gelenkeinrichtung 30 an dem Oberteil 10 befestigt werden können. Durch die Vielzahl von Befestigungseinrichtungen 76 an dem Oberteil 10 oder der kraftübertragenden Schnittstelle 51 können unterschiedliche Aktuatormodelle mit unterschiedlichen Verlagerungswegen und Bewegungsumfängen an der orthopädietechnischen Einrichtung festgelegt werden.

In der mittleren Darstellung der Figur 8 ist eine Variante des Adapters 70 gezeigt, bei der mehrere Befestigungselemente 72, 72', 72" auf einer Kreisbahn angeordnet, deren Mittelpunkt mit der Gelenkachse 40 zusammenfällt. Dadurch ist es möglich, eine Winkelverstellung durch eine Verdrehung um die Gelenkachse 40 herum zu erreichen. Werden beispielsweise alle 5° Befestigungselemente 72, 72' und 72" an dem Adapter 70 angeordnet oder ausgebildet, kann durch ein versetztes Montieren des Adapters 70 an dem Unterteil 20 oder der Schnittstelle 52 eine Abstufung des Extensionswinkelanschlages um jeweils 5° erreicht werden.

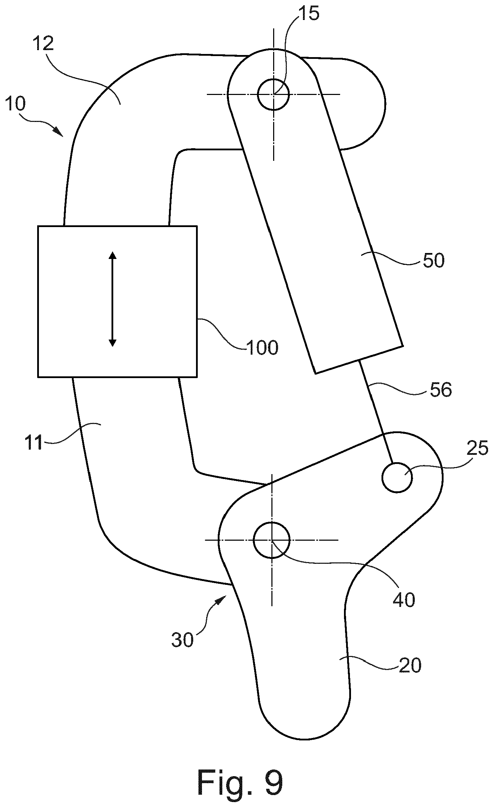

Eine weitere Variante der Erfindung ist in der Figur 9 gezeigt, bei der das Oberteil 10 zweigeteilt ausgebildet ist. Statt eines Oberteil 10 kann eine entsprechende Ausgestaltung auch für ein Unterteil 20 oder eine kraftübertragende Schnittstelle 51, 52 ausgeführt werden. Ein erstes Teil 11 weist eine Aufnahme für das Unterteil 20 mit der Gelenkachse 40 zur Ausbildung der Gelenkeinrichtung 30 auf. Das zweite Teil 12 weist die Befestigungsstelle 15 für den Aktuator 50 auf. Zwischen dem ersten Teil 11 und dem zweiten Teil 12 ist eine Verstelleinrichtung 100 angeordnet, über die die beiden Teile 11, 12 zueinander verschoben werden können. Beispielsweise kann eine geradlinige Verschiebung über eine Gewindelösung, eine teleskopierbare Ausgestaltung oder aber auch eine Verdrehung oder eine Verlagerung über einen Exzenter durch die Verstelleinrichtung 100 erfolgen, sodass die Befestigungsstelle 15 von der Gelenkachse 40 weg verlagert oder hin verlagert wird. Durch die entsprechende Verlagerung der Befestigungsstelle 15 von oder zu der Gelenkachse 40 wird, wie oben beschrieben, der maximale Extensionswinkel zwischen dem Oberteil 10 und dem Unterteil 20 oder zwischen dem Oberteil 10 und der kraftübertragenden Schnittstelle 52 verändert. Statt einer Ausgestaltung des Oberteils 10 aus zwei Teilen 11, 12, kann auch die kraftübertragende Schnittstelle 51 zweiteilig ausgebildet sein, gleiches gilt für das Unterteil 20 oder die zweite kraftübertragende Schnittstelle 52. Somit wird durch die Längenveränderung des Oberteils 10, des Unterteils 20 oder einer der kraftübertragenden Schnittstellen 51, 52 eine Veränderung des Extensionsanschlages und damit eine Veränderung der maximal erreichbaren Orientierung zwischen dem Oberteil 10 und dem Unterteil 20 und damit einer Gliedmaße und dem Unterteil 20 erreicht.

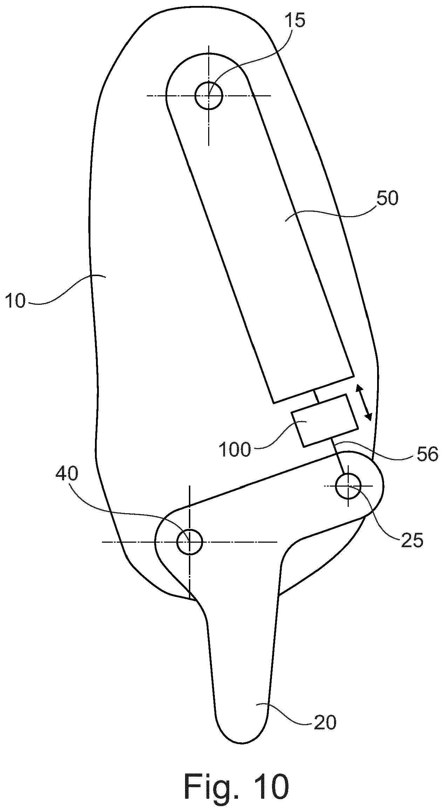

Eine weitere Variante ist in der Figur 10 dargestellt. Der grundsätzliche Aufbau entspricht dem der Figur 3. Statt der Verlagerung der Befestigungsstelle 15 ist in dieser Variante vorgesehen, dass die Kolbenstange 56 längenveränderlich ausgebildet ist. Dazu ist eine Verstelleinrichtung 100 in der Kolbenstange 56 integriert, um deren Länge zu verändern. Durch die Veränderung der Länge der Kolbenstange 56 wird der maximale Extensionswinkel verändert und damit der Extensionsanschlag eingestellt.

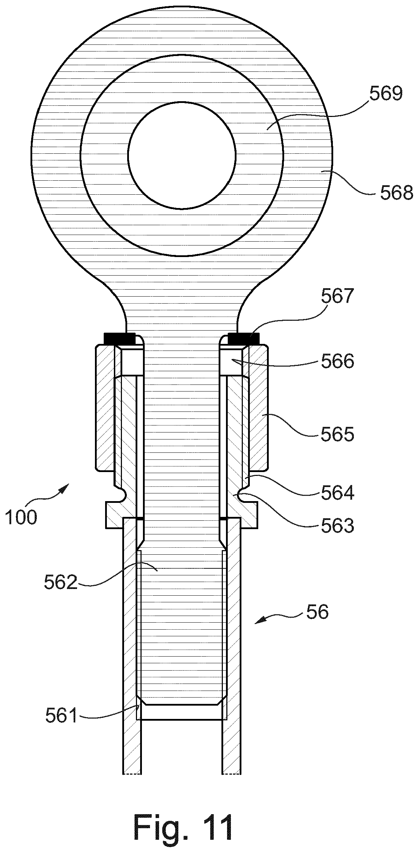

Die Figur 11 zeigt ein Ausführungsbeispiel einer längenveränderlichen Kolbenstange 56 in einer Schnittdarstellung. Innerhalb der Kolbenstange 56 ist ein Innengewinde 561 angeordnet, das mit einem Schraubeinsatz 562 wechselwirkt. Der Schraubeinsatz 562 als Bestandteil der Kolbenstange 56 kann bis zum Ende des Innengewindes 561 eingeschraubt werden, um die minimale Länge der Kolbenstange 56 mit dem Schraubeinsatz 562, an dem eine Lageraufnahme 568 mit einem Schwenklager 569 angeordnet ist, auszubilden. Zur Verlängerung der Kolbenstange 56 wird der Schraubeinsatz 562 herausgedreht. Zur Sicherung der eingenommenen Stellung ist zwischen der Lageraufnahme 568 und der Kolbenstange 56 eine Verstelleinrichtung 100 mit zwei Schraubhülsen 563, 565 angeordnet. Die untere Schraubhülse 563 weist ein Außengewinde 564 auf, das mit einem Innengewinde 566 der äußeren Schraubhülse 565 wechselwirkt. Das obere Ende der äußeren Schraubhülse 565 stützt sich über eine Tragscheibe 567 an der Lageraufnahme 568 ab, das untere Ende der inneren Schraubhülse 563 stützt sich an der Kolbenstange 56 ab. Zur Sicherung der einmal eingestellten Position des Schraubeinsatzes 562 sowie zum Aufbau einer Vorspannung im Schraubeinsatz 562 werden die Schraubhülsen 563, 565 so gegeneinander verdreht, dass die jeweiligen Enden gegen die Kolbenstange 56 bzw. die Tragscheibe 567 verspannt werden, wodurch eine Verdrehung des Schraubeinsatzes 562 relativ zu der Kolbenstange 56 verhindert wird. Durch die Verlängerung der Kolbenstange 56 bzw. des Herausschraubens des Schraubeinsatzes 562 wird eine Ausgestaltung gemäß Figur 10 eine Verschwenkung des Unterteils 20 im Uhrzeigersinn über einen größeren Winkelbereich ermöglicht, sodass der Extensionsanschlag weiter nach vorne verlagert wird, also in Richtung der maximalen Streckung.

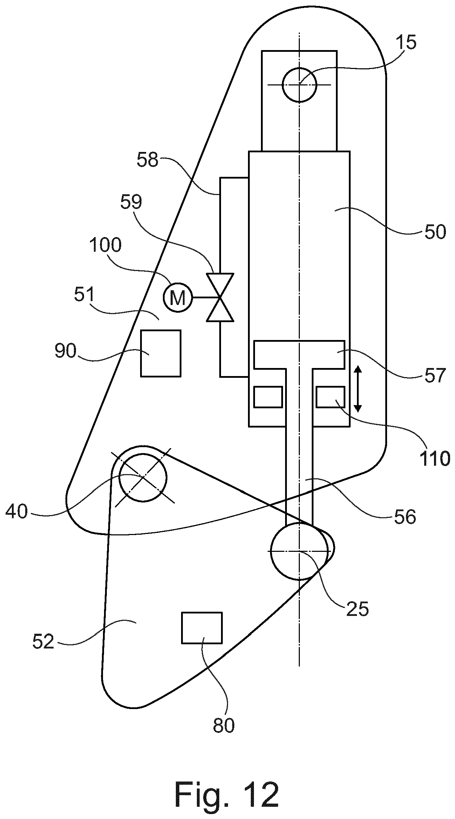

Varianten der Einrichtung sind in der Figur 12 gezeigt, bei der neben einem mechanischen Extensionsanschlag 110, der innerhalb des Aktuators 50 in dem Zylinder angeordnet ist, weitere Möglichkeiten zur Einstellung des Extensionsanschlages gezeigt sind. Der mechanische Extensionsanschlag 110 kann, wie durch den Doppelpfeil angedeutet, in Längserstreckung der Kolbenstange 56 innerhalb des Zylinders in dem Aktuator 5 verlagert werden, sodass der Kolben 57 an dem mechanischen Extensionsanschlag 110 anschlägt. Der Anschlag ist einstellbar. Wird der Extensionsanschlag 110 nach oben verlagert, wird der Extensionsanschlag 110 früh erreicht, das Bein oder der Arm wird in einer flektierten Stellung an einer weiteren Extension gehindert. Wird der Extensionsanschlag 110 weiter nach unten in Richtung auf die untere Befestigungsstelle 25 verlagert, vergrößert sich der Verschwenkwinkel in Extensionsrichtung um die Gelenkachse 40. Alternativ können auch Außengewinde 564 und Innengewinde 566 zwischen den beiden Komponenten 563 und 565 vertauscht sein.

Alternativ oder ergänzend zu dem mechanischen Extensionsanschlag 110 kann in einer Hydraulikleitung 58, die von einer Extensionskammer zu einer Flexionskammer führt, ein Stellventil 59 angeordnet sein, das motorisch angetrieben wird, sodass der Stellmotor oder die Stelleinrichtung die Verstelleinrichtung 100 ausbildet, die mit einer Steuerungseinrichtung 90 gekoppelt ist. Über die Steuerungseinrichtung 90 wird in Abhängigkeit von einem oder mehrerer Messwerte, beispielsweise von einem gemessenen Gelenkwinkel und/oder einem übertragenen Moment oder Interaktionskraft, die bspw. über einen Sensor 80 oder mehrere Sensoren 80 erfasst werden, das Hydraulikventil 59 geöffnet oder geschlossen. Soll bspw. bei einem Gelenkwinkel von 170° der Extensionsanschlag erfolgen, wird bei Erreichen eines entsprechenden Winkels oder einer entsprechenden Orientierung des Oberteils 10 relativ zum Unterteil 20 bzw. der ersten kraftübertragenden Schnittstelle 51 zu der zweiten kraftübertragenden Schnittstelle 52 das Hydraulikventil 59 geschlossen. Eine Weiterverlagerung des Kolbens 57 innerhalb des Zylinders in dem Hydraulikaktuator 50 wird verhindert, sodass effektiv ein Extensionsanschlag ausgebildet wird. Neben einer Ausgestaltung des Sensors 80 als ein Winkelsensor kann dieser auch als Kraft- oder Momentensensor oder als Sensor zur Erfassung der Orientierung eines Oberteils oder Unterteils im Raum oder eine beliebige Kombination dieser Sensoren ausgebildet sein. Zur Erfassung der Raumorientierung ist ein sogenannter Inertialwinkelsensor vorgesehen, der an dem Oberteil oder dem Unterteil angeordnet sein kann. Es ist auch möglich, über zwei Inertialwinkelsensoren die Relativlage des Oberteils zu dem Unterteil zu ermitteln und ein entsprechendes Steuerungssignal von der Steuerungseinrichtung 90 an die Verstelleinrichtung 100 zu senden, um das Ventil 59 zu öffnen oder zu schließen.

Sowohl die hydraulische Ausgestaltung des Extensionsanschlages 110 als auch die mechanische Ausgestaltung des Extensionsanschlages 110 innerhalb eines Aktuators als auch die Verlagerung der Befestigungsstellen 15, 25 an dem Oberteil 10 und/oder Unterteil bzw. einer kraftübertragenden Schnittstelle 51, 52 oder einem Adapter 70 können miteinander kombiniert werden, wobei beliebige Kombinationen der jeweiligen Verstelleinrichtungen und Verstellmechanismen miteinander möglich sind.

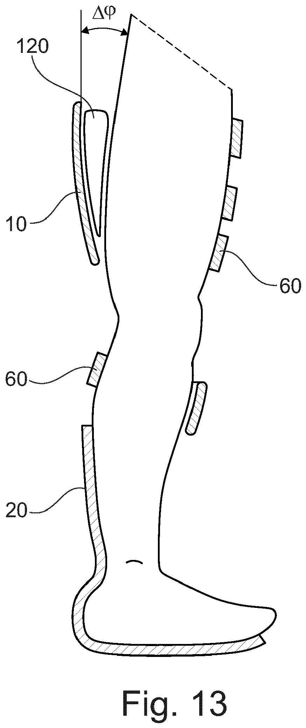

Eine weitere Variante der Einrichtung ist in der Figur 13 dargestellt, bei der die orthopädietechnische Einrichtung ebenfalls als Orthese ausgebildet ist. Das Oberteil 10 ist als Oberschenkelschale ausgebildet und über Befestigungseinrichtungen 60 in Gestalt von Spangen oder Gurten an dem Oberschenkel festgelegt. Über eine nicht dargestellte Gelenkeinrichtung ist das Unterteil 20 mit einer Fußaufnahme und einer Schienenbeinanlage über eine Befestigungseinrichtung 60 an dem Unterschenkel festgelegt. Zur Veränderung der Orientierung des Unterteils 20 relativ zu dem Oberschenkel als dem an dem Oberteil 10 festlegbaren Gliedmaße ist an der Rückseite des Oberschenkels an dem Oberteil 10 eine Anlage 120 in Gestalt eines Keilkissens oder eines Polsters angeordnet. Die posteriore Anlage des Oberschenkels an dem Oberteil 10 wird durch die Anlage 120 einstellbar. Durch Veränderung der Anlage 120, beispielsweise durch Auswechseln der Anlage 120 gegen eine schmalere Anlage 120, wird eine Veränderung der Position der oberen Gliedmaße relativ zu dem Unterteil 20 bewirkt. Die Veränderung des Winkels ? zwischen dem Oberteil 10 und der Gliedmaße kann entweder durch Auswechseln der Anlage 120 oder aber durch Aufpumpen eines Polsters oder durch Umpumpen eines Fluides in einer hohl ausgebildeten Anlage 120 stattfinden. Ebenfalls ist es möglich, eine Anlage 120 anterior der Gliedmaße anzuordnen, gegebenenfalls in Verbindung mit einer posterioren Anlage 120, sodass durch Umpumpen von Fluid aus der posterioren Anlage 120 zu einer anterioren Anlagen 120 die Orientierung des Oberschenkels relativ zu dem Unterteil 20 und damit eine Veränderung des Extensionsanschlages bewirkt werden kann. Alternativ oder ergänzend können ähnliche Einstellungen oder Modifikationen auch im Bereich des Unterschenkels durchgeführt werden.

Neben einer Verlagerung der Befestigungsstellen 15, 25 als solchen ist es möglich, die Länge des Aktuatorgehäuses 55 zu verändern, indem dieses beispielsweise über eine Schraubhülse längenveränderlich ausgestaltet ist, ähnlich einer Längenveränderung der Kolbenstange 57, wie in der Figur 11 beschrieben.

Durch eine orthopädietechnische Einrichtung 1, wie sie anhand der vorherigen Figuren beschrieben worden ist, ist es möglich, den effektiven Bewegungsumfang des Oberteils 10 relativ zum Unterteil 20 um die Gelenkachse 40 zu verändern. Der Bewegungsumfang des Aktuators 50, der rein passiv oder auch als Antrieb ausgebildet sein kann, wird beeinflusst, beispielsweise durch eine Verstellung eines mechanischen Extensionsanschlages, durch Verlagerung der Position des Aktuators, durch eine Verstellung von Befestigungsstellen 15, 25 zueinander bzw. zu der Gelenkachse oder durch Öffnen oder Schließen von Hydraulikventilen oder Hydraulikanschlägen. Die Veränderung des Extensionsanschlages, unabhängig von der Art und Weise, wie der Extensionsanschlag ausgebildet ist, kann beispielsweise auf Basis des Gelenkwinkels und des gemessenen Momentes um die Gelenkachse 40 erfolgen. Über das gemessene Moment und den gemessenen Winkel kann detektiert werden, ob die Gelenkeinrichtung 30 den vorgegebenen Gelenkwinkel mit der vorgegebenen Kraft erreicht. Bei einer Ausgestaltung der orthopädietechnischen Einrichtung als Orthese kann darüber zum Beispiel bestimmt werden, wie groß die Muskelaktivität der versorgten Gliedmaße bzw. des versorgten Körperteils ist. Beispielsweise kann bestimmt werden, ob eine ausreichende Streckung durch die Oberschenkelstreckmuskulatur aufgebracht wird, um das Unterteil in den Extensionsanschlag zu bewegen. Ein solches Erreichen des Extensionsanschlages kann als weiteres Steuerungssignal verwendet werden. Mit der beschriebenen orthopädietechnischen Einrichtung ist es möglich, eine Anpassbarkeit der orthopädietechnischen Einrichtung an zeitliche Änderungen der Anforderungen an die orthopädietechnische Einrichtung zu ermögliche, beispielsweise um veränderte anatomische und physiologische Bedingungen zu erfassen und einen Extensionsanschlag zu verstellen, wenn im Laufe des Benutzens der orthopädietechnischen Einrichtung eine Beugekontraktion nachlässt oder wenn detektiert wird, dass über einen gewissen Zeitraum, der in der Steuerung abgelegt ist, der Extensionsanschlag bei ansonsten unveränderten Bewegungsmustern nicht erreicht wird. Das kann darauf hindeuten, dass eine Schädigung vorliegt, eine Ermüdung eingetreten ist oder eine sonstige Problematik auftritt, auf die im Verlauf der Benutzung der orthopädietechnischen Einrichtung reagiert werden muss und mit der beschriebenen orthopädietechnischen Einrichtung auch reagiert werden kann. Die Veränderung des Bewegungsumfanges, insbesondere des Extensionsanschlages und damit der Position des Unterteils relativ zu dem Oberteil, kann automatisch erfolgen, wenn bestimmte Kriterien erfüllt sind oder nicht erfüllt werden. Es ergibt sich somit eine sich selbstständig anpassende orthopädietechnische Einrichtung.

Die Sensordaten sind nicht auf Winkeldaten oder Raumlagedaten beschränkt, sondern können auch Kräfte oder Momente erfassen. Mehrere Sensoren können der orthopädietechnischen Einrichtung angeordnet sein. Ebenso ist es möglich, dass physiologische Daten, beispielsweise elektromyographische Sensorsignale erfasst und zur Steuerung der orthopädietechnischen Einrichtung zur Verstellung des Extensionsanschlages oder der Orientierung einer Gliedmaße relativ zu einem Unterteil verwendet wird. Über die vorgegebenen Steuerungsdaten ist es möglich, den Extensionsanschlag autoadaptiv zu verstellen, ohne dass ein Patient oder ein Orthopädietechniker eine Verstellung ausführen muss. Die autoadaptive Verstellung erfolgt über die Verstelleinrichtung, die angetrieben ist und in Abhängigkeit von Sensordaten die orthopädietechnische Einrichtung und den jeweils anzustrebenden Extensionswinkel einstellt.

## Patentansprüche

1. Orthopädietechnische Einrichtung mit einem Oberteil (10) und einem Unterteil (20), die über zumindest eine Gelenkeinrichtung (30) schwenkbar um eine Gelenkachse (40) miteinander verbunden sind und zumindest eine Befestigungseinrichtung (60) aufweist, mit denen die orthopädietechnische Einrichtung an einer Gliedmaße festlegbar ist, und mit einem Aktuator (50), der an dem Oberteil (10) und dem Unterteil (20) an Befestigungsstellen (15, 25) festgelegt ist und eine Verschwenkung des Oberteils (10) relativ zu dem Unterteil (20) beeinflusst, wobei die Orientierung des Unterteils (20) relativ zu der an dem Oberteil (10) festlegbaren Gliedmaße einstellbar ist, **dadurch gekennzeichnet, dass** die Befestigungsstelle (15, 25) verschieblich oder verdrehbar an dem Oberteil (10) oder Unterteil (20) oder einer kraftübertragenden Schnittstelle (51, 52) gelagert ist oder die Befestigungsstelle (15, 25) an einer auswechselbaren Komponente (170) angeordnet oder ausgebildet ist, um unterschiedliche Positionen der Befestigungsstelle (15, 25) für den Aktuator (50) zu realisieren.

2. Orthopädietechnische Einrichtung nach Anspruch 1, **dadurch gekennzeichnet, dass** an der orthopädietechnischen Einrichtung (1) ein einstellbarer Extensionsanschlag (110) und/oder Flexionsanschlag angeordnet oder ausgebildet und/oder eine auswechselbare oder einstellbare Anlage (120) für die Gliedmaße angeordnet ist.

3. Orthopädietechnische Einrichtung nach Anspruch 2, **dadurch gekennzeichnet, dass** der Extensionsanschlag (110) an oder in dem Aktuator (50), an oder in einer kraftübertragenden Schnittstelle (51, 52) zwischen dem Aktuator (50) und dem Oberteil (10) und/oder Unterteil (20) und/oder an oder in der zumindest einen Gelenkeinrichtung (30) ausgebildet ist.

4. Orthopädietechnische Einrichtung nach einem der voranstehenden Ansprüche, **dadurch gekennzeichnet, dass** der Abstand zumindest einer Befestigungsstelle (15, 25) zu der Gelenkachse (40) verstellbar ist.

5. Orthopädietechnische Einrichtung nach einem der voranstehenden Ansprüche, **dadurch gekennzeichnet, dass** zumindest eine der Befestigungsstellen (15, 25) verlagerbar an dem jeweils zugeordneten Oberteil (10) oder Unterteil (20) oder einer kraftübertragenden Schnittstelle (51, 52) gelagert ist.

6. Orthopädietechnische Einrichtung nach einem der voranstehenden Ansprüche, **dadurch gekennzeichnet, dass** die Befestigungsstelle (15, 25) motorisch verstellbar an dem Oberteil (10) oder Unterteil (20) oder einer kraftübertragenden Schnittstelle (51, 52) gelagert ist.

7. Orthopädietechnische Einrichtung nach einem der voranstehenden Ansprüche, **dadurch gekennzeichnet, dass** die Befestigungsstelle (15, 25) an einem Adapter (70) angeordnet oder ausgebildet ist, an dem mehrere Befestigungseinrichtungen (72) für den Aktuator (50) angeordnet sind.

8. Orthopädietechnische Einrichtung nach einem der Ansprüche 1 bis 6, **dadurch gekennzeichnet, dass** die Befestigungsstelle (15, 25) oder Gelenkeinrichtung (30) an einem auswechselbaren Adapter (70) angeordnet oder ausgebildet ist.

9. Orthopädietechnische Einrichtung nach einem der voranstehenden Ansprüche, **dadurch gekennzeichnet, dass** das Oberteil (10) und/oder das Unterteil (20) und/oder eine kraftübertragende Schnittstelle (51, 52) und/oder der Aktuator (50) längenveränderlich ausgebildet sind.

10. Orthopädietechnische Einrichtung nach einem der voranstehenden Ansprüche, **dadurch gekennzeichnet, dass** das Oberteil (10) und/oder Unterteil (20) und/oder eine kraftübertragende Schnittstelle (51, 52) mehrteilig ausgebildet sind, an einem ersten Teil (11) die Gelenkeinrichtung (30) ausgebildet und an einem zweiten Teil (12) die Befestigungsstelle (15, 25) angeordnet ist und das erste Teil (11) verschieblich oder verdrehbar zu dem zweiten Teil (12) mit dem zweiten Teil (12) verbunden ist.

11. Orthopädietechnische Einrichtung nach Anspruch 10, **dadurch gekennzeichnet, dass** der Aktuator (50) ein längenveränderliches Gehäuse (55) und/oder eine längenveränderliche Kolbenstange (56) aufweist.

12. Orthopädietechnische Einrichtung nach einem der voranstehenden Ansprüche, **dadurch gekennzeichnet, dass** zumindest ein Sensor (80) zur Erfassung der zwischen dem Oberteil (10) und dem Unterteil (20) übertragenen Kraft und/oder des übertragenen Momentes, des Gelenkwinkels, der Raumlage des Oberteils (10) und/oder des Unterteils (20) und/oder biometrischer Signale eines Nutzers der orthopädietechnischen Einrichtung zugeordnet ist.

13. Orthopädietechnische Einrichtung nach einem der voranstehenden Ansprüche, **dadurch gekennzeichnet, dass** der Aktuator (50) als Hydraulikaktuator ausgebildet ist.

14. Orthopädietechnische Einrichtung nach Anspruch 13, **dadurch gekennzeichnet, dass** der Extensionsanschlag (110) durch Schließen eines Ventils und/oder Blockieren einer hydraulischen Leitung realisiert wird.

15. Orthopädietechnische Einrichtung nach einem der voranstehenden Ansprüche, **dadurch gekennzeichnet, dass** sie als steuerbare Einrichtung ausgebildet und mit zumindest einem Sensor (80) versehen ist, der mit einer Steuerungseinrichtung (90) gekoppelt ist, die eine Verstelleinrichtung (100) zur Verstellung eines Extensionsanschlages (110), der einen maximalen Gelenkwinkel begrenzt, aktiviert oder deaktiviert, wobei der Extensionsanschlag (110) in Abhängigkeit von Sensordaten von einer Ausgangsstellung, in der das Oberteil (10) relativ zu dem Unterteil (20) in einer flektierten Stellung befindlich ist, in eine Endstellung verlagert wird, in der sich das Oberteil (10) relativ zu dem Unterteil (20) in einer gegenüber der Ausgangsstellung veränderten Stellung befindet.

16. Orthopädietechnische Einrichtung nach Anspruch 15, **dadurch gekennzeichnet, dass** die Steuerungseinrichtung (90) ausgebildet ist, die Sensordaten über einen Zeitraum zu erfassen und mit einem Schwellwert zu vergleichen, wobei eine Verstellung des Extensionsanschlages (110) nur dann erfolgt, wenn der Schwellwert über einen definierten Zeitraum erreicht wird.

17. Orthopädietechnische Einrichtung nach Anspruch 15 oder 16, **dadurch gekennzeichnet, dass** der maximale Gelenkwinkel einstellbar ist und der Extensionsanschlag (110) in Abhängigkeit von dem gemessenen Gelenkwinkel bis zum maximalen Gelenkwinkel verstellbar ist.

18. Orthopädietechnische Einrichtung nach einem der Ansprüche 15 bis 17, **dadurch gekennzeichnet, dass** die Steuerung (90) eingerichtet ist, dass bei Erreichen des Extensionsanschlages (110) das um die Gelenksachse (40) wirksame Moment oder die zwischen dem Oberteil (10) und dem Unterteil (20) wirksame Kraft gemessen wird und bei Überschreiten eines Schwellwertes der Extensionsanschlag (110) in Richtung auf seine Endstellung verlagert wird und bei Unterschreiten des Schwellwertes der Extensionsanschlag (110) in entgegengesetzte Richtung verlagert wird.

19. Orthopädietechnische Einrichtung nach einem der Ansprüche 15 bis 18, **dadurch gekennzeichnet, dass** die Steuerung (90) eingerichtet ist, Widerstandswerte des Aktuators (50) während der Verschwenkbewegung zu erfassen und mit Sensorwerten bezüglich des Gelenkwinkels und der zwischen dem Oberteil (10) und dem Unterteil (20) herrschenden Kräfte und/oder Momente zu korrelieren und bei einer Abweichung eines Kräfte- und Momentenverlaufs von dem Widerstandsverlaufs eine Verstellung des Extensionsanschlages (110) durchzuführen.

20. Orthopädietechnische Einrichtung nach einem der Ansprüche 15 bis 19, **dadurch gekennzeichnet, dass** der Extensionsanschlag autoadaptiv verstellbar ist.

## Claims

1. An orthopedic device with an upper part (10) and a lower part (20), which are connected to each other by at least one joint device (30) so as to be pivotable about a joint axis (40) and has at least one fastening device (60) with which the orthopedic device can be secured to a limb, and with an actuator (50), which is secured to the upper part (10) and the lower part (20) at fastening points (15, 25) and influences a pivoting of the upper part (10) relative to the lower part (20), wherein the orientation of the lower part (20) is adjustable relative to the limb that can be secured to the upper part (10), **characterized in that** the fastening point (15, 25) is mounted displaceably or rotatably on the upper part (10) or lower part (20) or on a force-transmitting interface (51, 52) or that the fastening point (15, 25) is arranged or formed on an exchangeable component (170), in order to realize different positions of the fastening point (15, 25) for the actuator (50).

2. The orthopedic device as claimed in claim 1, **characterized in that** an adjustable extension stop (110) and/or flexion stop is arranged or formed on the orthopedic device (1), and/or an exchangeable or adjustable support (120) for the limb is arranged thereon.

3. The orthopedic device as claimed in claim 2, **characterized in that** the extension stop (110) is formed on or in the actuator (50), on or in a force-transmitting interface (51, 52) between the actuator (50) and the upper part (10) and/or lower part (20), and/or on or in the at least one joint device (30).

4. The orthopedic device as claimed in one of the preceding claims, **characterized in that** the distance of at least one fastening point (15, 25) from the joint axis (40) is adjustable.

5. The orthopedic device as claimed in one of the preceding claims, **characterized in that** at least one of the fastening points (15, 25) is mounted displaceably on the respectively associated upper part (10) or lower part (20) or on a force-transmitting interface (51, 52).

6. The orthopedic device as claimed in one of the preceding claims, **characterized in that** the fastening point (15, 25) is mounted, adjustable by motor, on the upper part (10) or lower part (20) or on a force-transmitting interface (51, 52).

7. The orthopedic device as claimed in one of the preceding claims, **characterized in that** the fastening point (15, 25) is arranged or formed on an adapter (70) on which several fastening devices (72) for the actuator (50) are arranged.

8. The orthopedic device as claimed in one of claims 1 through 6, **characterized in that** the fastening point (15, 25) or joint device (30) is arranged or formed on an exchangeable adapter (70).

9. The orthopedic device as claimed in one of the preceding claims, **characterized in that** the upper part (10) and/or the lower part (20) and/or a force-transmitting interface (51, 52) and/or the actuator (50) are variable in length.

10. The orthopedic device as claimed in one of the preceding claims, **characterized in that** the upper part (10) and/or lower part (20) and/or a force-transmitting interface (51, 52) have a multi-part design, the joint device (30) is formed on a first part (11) and the fastening point (15, 25) is arranged on a second part (12), and the first part (11) is connected to the second part (12) so as to be displaceable or rotatable with respect to the second part (12) .

11. The orthopedic device as claimed in claim 10, **characterized in that** the actuator (50) has a housing (55) of variable length and/or a piston rod (56) of variable length.

12. The orthopedic device as claimed in one of the preceding claims, **characterized in that** at least one sensor (80) for detecting the force transmitted between the upper part (10) and the lower part (20) and/or the transmitted moment, the joint angle, the spatial position of the upper part (10) and/or of the lower part (20), and/or biometric signals of a user, is assigned to the orthopedic device.

13. The orthopedic device as claimed in one of the preceding claims, **characterized in that** the actuator (50) is designed as a hydraulic actuator.

14. The orthopedic device as claimed in claim 13, **characterized in that** the extension stop (110) is realized by closing a valve and/or blocking a hydraulic line.

15. The orthopedic device as claimed in one of the preceding claims, **characterized in that** it is designed as a controllable device and is provided with at least one sensor (80), which is coupled to a controller (90) which activates or deactivates an adjustment device (100) for adjusting an extension stop (110) that limits a maximum joint angle, wherein the extension stop (110), in accordance with sensor data, is moved from a starting position, in which the upper part (10) is located in a position of flexion relative to the lower part (20), to an end position, in which the upper part (10) is located, relative to the lower part (20), in a position different than the starting position.

16. The orthopedic device as claimed in claim 15, **characterized in that** the controller (90) is configured to detect the sensor data over a period of time and to compare them to a threshold value, wherein an adjustment of the extension stop (110) takes place only when the threshold value is reached over a defined period of time.

17. The orthopedic device as claimed in claim 15 or 16, **characterized in that** the maximum joint angle is adjustable, and, in accordance with the measured joint angle, the extension stop (110) is adjustable as far as the maximum joint angle.

18. The orthopedic device as claimed in one of claims 15 through 17, **characterized in that** the controller (90) is configured such that, when the extension stop (110) is reached, the moment effective about the joint axis (40) or the force effective between the upper part (10) and the lower part (20) is measured, and, if a threshold value is exceeded, the extension stop (110) is moved in the direction of its end position, and, if the threshold value is not reached, the extension stop (110) is moved in the opposite direction.

19. The orthopedic device as claimed in one of claims 15 through 18, **characterized in that** the controller (90) is configured to detect resistance values of the actuator (50) during the pivoting movement and to correlate them with sensor values concerning the joint angle and the forces and/or moments prevailing between the upper part (10) and the lower part (20), and, if a force and moment profile deviates from the resistance profile, to perform an adjustment of the extension stop (110).

20. The orthopedic device as claimed in one of claims 15 through 19, **characterized in that** the extension stop is adjustable auto-adaptively.

## Revendications

1. Dispositif orthopédique comprenant une partie supérieure (10) et une partie inférieure (20) qui sont reliées entre elles par au moins un dispositif d'articulation (30) de manière à pouvoir pivoter autour d'un axe d'articulation (40) et qui présentent au moins un dispositif de fixation (60) permettant d'immobiliser le dispositif orthopédique sur un membre, et comprenant un actionneur (50) qui est immobilisé sur la partie supérieure (10) et sur la partie inférieure (20) en des points de fixation (15, 25) et qui influence un pivotement de la partie supérieure (10) par rapport à la partie inférieure (20), l'orientation de la partie inférieure (20) par rapport au membre immobilisable sur la partie supérieure (10) étant réglable, **caractérisé en ce que** le point de fixation (15, 25) est monté de façon mobile en translation ou en rotation sur la partie supérieure (10) ou sur la partie inférieure (20) ou sur une interface de transmission de force (51, 52), ou le point de fixation (15, 25) est disposé ou réalisé sur un composant interchangeable (170) afin de réaliser différentes positions du point de fixation (15, 25) pour l'actionneur (50).

2. Dispositif orthopédique selon la revendication 1, **caractérisé en ce qu'**une butée d'extension (110) et/ou une butée de flexion réglable est disposée ou réalisée sur le dispositif orthopédique (1), et/ou un appui (120) interchangeable ou réglable pour le membre est disposé sur ledit dispositif.

3. Dispositif orthopédique selon la revendication 2, **caractérisé en ce que** la butée d'extension (110) est réalisée sur ou dans l'actionneur (50), sur ou dans une interface de transmission de force (51, 52) entre l'actionneur (50) et la partie supérieure (10) et/ou la partie inférieure (20), et/ou sur ou dans ledit au moins un dispositif d'articulation (30).

4. Dispositif orthopédique selon l'une des revendications précédentes, **caractérisé en ce que** la distance d'au moins un point de fixation (15, 25) par rapport à l'axe d'articulation (40) est ajustable.

5. Dispositif orthopédique selon l'une des revendications précédentes, **caractérisé en ce que** l'un au moins des points de fixation (15, 25) est monté de manière déplaçable sur la partie supérieure (10) ou la partie inférieure (20) respectivement associée ou sur une interface de transmission de force (51, 52).

6. Dispositif orthopédique selon l'une des revendications précédentes, **caractérisé en ce que** le point de fixation (15, 25) est monté sur la partie supérieure (10) ou la partie inférieure (20) ou sur une interface de transmission de force (51, 52) de manière à pouvoir être déplacé par voie motrice.

7. Dispositif orthopédique selon l'une des revendications précédentes, **caractérisé en ce que** le point de fixation (15, 25) est disposé ou réalisé sur un adaptateur (70), sur lequel sont disposés plusieurs dispositifs de fixation (72) pour l'actionneur (50).

8. Dispositif orthopédique selon l'une des revendications 1 à 6, **caractérisé en ce que** le point de fixation (15, 25) ou le dispositif d'articulation (30) est disposé ou réalisé sur un adaptateur interchangeable (70).

9. Dispositif orthopédique selon l'une des revendications précédentes, **caractérisé en ce que** la partie supérieure (10) et/ou la partie inférieure (20) et/ou une interface de transmission de force (51, 52) et/ou l'actionneur (50) sont de longueur variable.

10. Dispositif orthopédique selon l'une des revendications précédentes, **caractérisé en ce que** la partie supérieure (10) et/ou la partie inférieure (20) et/ou une interface de transmission de force (51, 52) sont réalisées en plusieurs parties, le dispositif d'articulation (30) est réalisé sur une première partie (11) et le point de fixation (15, 25) est disposé sur une deuxième partie (12), et la première partie (11) est reliée à la deuxième partie (12) de façon mobile en translation ou en rotation par rapport à la deuxième partie (12).

11. Dispositif orthopédique selon la revendication 10, **caractérisé en ce que** l'actionneur (50) comprend un boîtier (55) de longueur variable et/ou une tige de piston (56) de longueur variable.

12. Dispositif orthopédique selon l'une des revendications précédentes, **caractérisé en ce qu'**au moins un capteur (80) est associé au dispositif orthopédique pour détecter la force et/ou le couple transmis(e) entre la partie supérieure (10) et la partie inférieure (20), l'angle d'articulation, la position spatiale de la partie supérieure (10) et/ou de la partie inférieure (20) et/ou des signaux biométriques d'un utilisateur.

13. Dispositif orthopédique selon l'une des revendications précédentes, **caractérisé en ce que** l'actionneur (50) est réalisé sous forme d'actionneur hydraulique.

14. Dispositif orthopédique selon la revendication 13, **caractérisé en ce que** la butée d'extension (110) est mise en oeuvre par la fermeture d'une valve et/ou par le blocage d'une conduite hydraulique.

15. Dispositif orthopédique selon l'une des revendications précédentes, **caractérisé en ce qu'**il est réalisé sous forme de dispositif contrôlable et est pourvu d'au moins un capteur (80) qui est couplé à un dispositif de commande (90) qui active ou désactive un dispositif de déplacement (100) pour déplacer une butée d'extension (110) limitant un angle d'articulation maximal, la butée d'extension (110) étant déplacée, en fonction de données de capteur, depuis une position initiale, dans laquelle la partie supérieure (10) se trouve dans une position fléchie par rapport à la partie inférieure (20), jusque dans une position finale, dans laquelle la partie supérieure (10) se trouve dans une position, modifiée par rapport à la position initiale, par rapport à la partie inférieure (20).

16. Dispositif orthopédique selon la revendication 15, **caractérisé en ce que** le dispositif de commande (90) est réalisé pour saisir les données de capteur sur une période de temps et pour les comparer à une valeur seuil, un déplacement de la butée d'extension (110) n'ayant lieu que si la valeur seuil est atteinte sur une période de temps définie.

17. Dispositif orthopédique selon la revendication 15 ou 16, **caractérisé en ce que** l'angle d'articulation maximal est réglable, et la butée d'extension (110) est déplaçable jusqu'à l'angle d'articulation maximal en fonction de l'angle d'articulation mesuré.

18. Dispositif orthopédique selon l'une des revendications 15 à 17, **caractérisé en ce que** la commande (90) est conçue de telle sorte que, lorsque la butée d'extension (110) est atteinte, le couple agissant autour de l'axe d'articulation (40) ou la force agissant entre la partie supérieure (10) et la partie inférieure (20) est mesuré(e) et, lorsqu'une valeur seuil est dépassée vers le haut, la butée d'extension (110) est déplacée en direction de sa position finale et, lorsque la valeur seuil est dépassée vers le bas, la butée d'extension (110) est déplacée dans la direction opposée.

19. Dispositif orthopédique selon l'une des revendications 15 à 18, **caractérisé en ce que** la commande (90) est conçue pour saisir des valeurs de résistance de l'actionneur (50) pendant le mouvement de pivotement et pour les corréler avec des valeurs de capteur concernant l'angle d'articulation et les forces et/ou couples régnant entre la partie supérieure (10) et la partie inférieure (20), et pour effectuer un déplacement de la butée d'extension (110) en cas d'écart entre une courbe de forces et de couples et la courbe de résistance.

20. Dispositif orthopédique selon l'une des revendications 15 à 19, **caractérisé en ce que** la butée d'extension est déplaçable de manière autoadaptative.
